(19)

Europäisches Patentamt
European Patent Office
Office européen des brevets

(11)  EP 2 426 204 A1

(12)  **EUROPEAN PATENT APPLICATION**

(43) Date of publication:
**07.03.2012  Bulletin 2012/10**

(21) Application number: **10175012.3**

(22) Date of filing: **02.09.2010**

(51) Int Cl.:
*C12N 15/82* (2006.01)        *A01H 5/00* (2006.01)
*C12N 15/29* (2006.01)

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**
Designated Extension States:
**BA ME RS**

(71) Applicant: **Ludwig-Maximilians-Universität
München
80359 München (DE)**

(72) Inventor: **The designation of the inventor has not
yet been filed**

(74) Representative: **Schiweck, Weinzierl & Koch
European Patent Attorneys
Landsberger Straße 98
80339 München (DE)**

(54)  **Spontaneous nodule organogenesis in plants**

(57)    The present invention provides a plant comprising a nucleotide sequence of a (mutant) CYCLOPS gene, wherein said nucleotide sequence comprises at a first position corresponding to position 148-150 of the nucleotide sequence shown in SEQ ID No:1 and/or at a second position corresponding to position 460-462 of the nucleotide sequence shown in SEQ ID No:1 a codon encoding an amino acid mimicking the effects of phosphorylation. Aso, plants are provided which comprise a CYCLOPS protein having at a first position corresponding to position 50 of the amino acid sequence of the CYCLOPS protein shown in SEQ ID No:2 and/or at a second position corresponding to position 154 of the amino acid sequence of the CYCLOPS protein shown in SEQ ID No:2 an amino acid mimicking the effects of phosphorylation. The plant of the present invention are capable of developing root nodules, preferably spontaneously developing nodules, preferably in the absence of rhizobia and/or rhizobial signal molecules.

EP 2 426 204 A1

SEQUENCE LISTING

<110> LMU
Ludwig-Maximilians-Universitaet Muenchen

<120> Spontaneous organogenesis in plants

<130> LMU13821EP

<160> 12

<170> PatentIn version 3.5

<210> 1
<211> 1557
<212> DNA
<213> Lotus japonicus

<220>
<221> CDS
<222> (1)..(1554)

<400> 1
```
atg gaa ggg agg ggg ttt tct ggt tta tat aga aac tca agt gaa gaa      48
Met Glu Gly Arg Gly Phe Ser Gly Leu Tyr Arg Asn Ser Ser Glu Glu
1               5                   10                  15

ttg ttc ctg aag aca gtg atg gag agc cct att ggt atg cca gtt cct      96
Leu Phe Leu Lys Thr Val Met Glu Ser Pro Ile Gly Met Pro Val Pro
                20                  25                  30

tca atg gag atg ctg ggt ttc aag aat gtt tct caa ggc ttt cgc gca     144
Ser Met Glu Met Leu Gly Phe Lys Asn Val Ser Gln Gly Phe Arg Ala
            35                  40                  45

gat agc gag gag ctt ttc aaa cgc tgg cta aca aat gga gag gga tac     192
Asp Ser Glu Glu Leu Phe Lys Arg Trp Leu Thr Asn Gly Glu Gly Tyr
        50                  55                  60

aat tca tca agc ata ggg ttt agc agt cga tta tca aag agg ata tcc     240
Asn Ser Ser Ser Ile Gly Phe Ser Ser Arg Leu Ser Lys Arg Ile Ser
65                  70                  75                  80

act gaa cta gtt aat gga tct aat cag cta caa gtt ggt gta gcc tca     288
Thr Glu Leu Val Asn Gly Ser Asn Gln Leu Gln Val Gly Val Ala Ser
                85                  90                  95

gat gga aga aac aat gac aaa cca ttc ata caa aat aac ctt ttg gca     336
Asp Gly Arg Asn Asn Asp Lys Pro Phe Ile Gln Asn Asn Leu Leu Ala
            100                 105                 110

aat gat gtt tca ggt gat ttc aat ttt cca atc aga gat cct gtt gat     384
Asn Asp Val Ser Gly Asp Phe Asn Phe Pro Ile Arg Asp Pro Val Asp
            115                 120                 125

aga gaa ctg caa cct agt aac ttg ttt cta gcc aag gcc tgg ttt ctc     432
```

```
Arg Glu Leu Gln Pro Ser Asn Leu Phe Leu Ala Lys Ala Trp Phe Leu
    130                 135                 140

agt gat caa cga atg aca aga agc cgg tcc tct gaa ttg cgg cgg cga    480
Ser Asp Gln Arg Met Thr Arg Ser Arg Ser Ser Glu Leu Arg Arg Arg
145                 150                 155                 160

tat tct gaa atg caa aat ggt cta gcc aca caa gga ata gaa tcc att    528
Tyr Ser Glu Met Gln Asn Gly Leu Ala Thr Gln Gly Ile Glu Ser Ile
                    165                 170                 175

tgc atg gat cct cag cat ggt gct gag gca aca aaa caa gaa gtt gca    576
Cys Met Asp Pro Gln His Gly Ala Glu Ala Thr Lys Gln Glu Val Ala
                180                 185                 190

aat ttc aat ggt tac aat tat ctc tct atg tgt gag ctt cca agt caa    624
Asn Phe Asn Gly Tyr Asn Tyr Leu Ser Met Cys Glu Leu Pro Ser Gln
                195                 200                 205

aag ggt tca ttc atg tct ccg tcc aac tca tgt tca tct aac ttc aac    672
Lys Gly Ser Phe Met Ser Pro Ser Asn Ser Cys Ser Ser Asn Phe Asn
    210                 215                 220

aca cct caa ttt ggc gac atg gat aaa gtt tca tct tgt gta agt atg    720
Thr Pro Gln Phe Gly Asp Met Asp Lys Val Ser Ser Cys Val Ser Met
225                 230                 235                 240

ctg aaa ggg aca tta caa cgc cgg aga ctc agc agt caa ctt gag aaa    768
Leu Lys Gly Thr Leu Gln Arg Arg Arg Leu Ser Ser Gln Leu Glu Lys
                    245                 250                 255

gaa gct gca gaa gat gac tta aat gga att ttt tat cct caa gaa cct    816
Glu Ala Ala Glu Asp Asp Leu Asn Gly Ile Phe Tyr Pro Gln Glu Pro
                260                 265                 270

ctt ttc caa act ggc ttt gat caa gga caa gaa aac tgg agt aat caa    864
Leu Phe Gln Thr Gly Phe Asp Gln Gly Gln Glu Asn Trp Ser Asn Gln
                275                 280                 285

acg cca gta aat gtt caa gta gac tct att ggt gaa gtt aag gat cat    912
Thr Pro Val Asn Val Gln Val Asp Ser Ile Gly Glu Val Lys Asp His
    290                 295                 300

gga gtc ctg caa aca cta gaa gga tcc aca aac cct gtc gtt gat ggt    960
Gly Val Leu Gln Thr Leu Glu Gly Ser Thr Asn Pro Val Val Asp Gly
305                 310                 315                 320

ttt gca aat cag ata aac caa atc tat gtc gga aca gct tct gga gaa   1008
Phe Ala Asn Gln Ile Asn Gln Ile Tyr Val Gly Thr Ala Ser Gly Glu
                325                 330                 335

cct tct caa agt gaa tcc tct aat gct gca cca gta atc tcc tct ggt   1056
Pro Ser Gln Ser Glu Ser Ser Asn Ala Ala Pro Val Ile Ser Ser Gly
                340                 345                 350

tta gac aca tgc gaa ggt ccc ata aac tcg aat caa act ctc tgc gaa   1104
Leu Asp Thr Cys Glu Gly Pro Ile Asn Ser Asn Gln Thr Leu Cys Glu
```

```
                   355                        360                        365

     agc tca tgg aaa caa gta gga gtg agt aaa agt tca gaa aat act caa    1152
     Ser Ser Trp Lys Gln Val Gly Val Ser Lys Ser Ser Glu Asn Thr Gln
         370                 375                 380

     aat aga gtc aaa ggt ttc aga gaa cag atc atg gat aat ctg aaa gat    1200
     Asn Arg Val Lys Gly Phe Arg Glu Gln Ile Met Asp Asn Leu Lys Asp
     385                 390                 395                 400

     gat aag aag aga aaa agt cta gaa aga tat gga tct ata aca tca gct    1248
     Asp Lys Lys Arg Lys Ser Leu Glu Arg Tyr Gly Ser Ile Thr Ser Ala
                         405                 410                 415

     gtt tca gat gac aag gga gac acc act aaa aag cgt agg gtg gaa cgc    1296
     Val Ser Asp Asp Lys Gly Asp Thr Thr Lys Lys Arg Arg Val Glu Arg
                     420                 425                 430

     tca agg aaa atg gct gaa gct aag gaa aga aat tcg aca cca tca gtt    1344
     Ser Arg Lys Met Ala Glu Ala Lys Glu Arg Asn Ser Thr Pro Ser Val
                 435                 440                 445

     ccc tca gat atg caa gct gtc ttg aag cgg tgc gaa aac ctt gag aag    1392
     Pro Ser Asp Met Gln Ala Val Leu Lys Arg Cys Glu Asn Leu Glu Lys
         450                 455                 460

     gaa gtt cga tcg cta aaa ctc aac ttg tcc ttc atg aat aga aag gat    1440
     Glu Val Arg Ser Leu Lys Leu Asn Leu Ser Phe Met Asn Arg Lys Asp
     465                 470                 475                 480

     tct gaa caa aca aag cag ata gaa gac ctt cag aag cag aat gaa gag    1488
     Ser Glu Gln Thr Lys Gln Ile Glu Asp Leu Gln Lys Gln Asn Glu Glu
                         485                 490                 495

     ctg gca gat gaa aaa gag cgc ctc ctc gaa gag att gaa aga att cta    1536
     Leu Ala Asp Glu Lys Glu Arg Leu Leu Glu Glu Ile Glu Arg Ile Leu
                 500                 505                 510

     tca gaa act gaa aaa atg taa                                        1557
     Ser Glu Thr Glu Lys Met
         515
```

```
<210>   2
<211>   518
<212>   PRT
<213>   Lotus japonicus

<400>   2

Met Glu Gly Arg Gly Phe Ser Gly Leu Tyr Arg Asn Ser Ser Glu Glu
1               5                   10                  15


Leu Phe Leu Lys Thr Val Met Glu Ser Pro Ile Gly Met Pro Val Pro
                20                  25                  30
```

```
Ser Met Glu Met Leu Gly Phe Lys Asn Val Ser Gln Gly Phe Arg Ala
        35                  40                  45

Asp Ser Glu Glu Leu Phe Lys Arg Trp Leu Thr Asn Gly Glu Gly Tyr
    50                  55                  60

Asn Ser Ser Ser Ile Gly Phe Ser Ser Arg Leu Ser Lys Arg Ile Ser
65                  70                  75                  80

Thr Glu Leu Val Asn Gly Ser Asn Gln Leu Gln Val Gly Val Ala Ser
                85                  90                  95

Asp Gly Arg Asn Asn Asp Lys Pro Phe Ile Gln Asn Asn Leu Leu Ala
            100                 105                 110

Asn Asp Val Ser Gly Asp Phe Asn Phe Pro Ile Arg Asp Pro Val Asp
        115                 120                 125

Arg Glu Leu Gln Pro Ser Asn Leu Phe Leu Ala Lys Ala Trp Phe Leu
    130                 135                 140

Ser Asp Gln Arg Met Thr Arg Ser Arg Ser Ser Glu Leu Arg Arg Arg
145                 150                 155                 160

Tyr Ser Glu Met Gln Asn Gly Leu Ala Thr Gln Gly Ile Glu Ser Ile
                165                 170                 175

Cys Met Asp Pro Gln His Gly Ala Glu Ala Thr Lys Gln Glu Val Ala
            180                 185                 190

Asn Phe Asn Gly Tyr Asn Tyr Leu Ser Met Cys Glu Leu Pro Ser Gln
        195                 200                 205

Lys Gly Ser Phe Met Ser Pro Ser Asn Ser Cys Ser Ser Asn Phe Asn
    210                 215                 220

Thr Pro Gln Phe Gly Asp Met Asp Lys Val Ser Ser Cys Val Ser Met
225                 230                 235                 240

Leu Lys Gly Thr Leu Gln Arg Arg Arg Leu Ser Ser Gln Leu Glu Lys
                245                 250                 255
```

Glu Ala Ala Glu Asp Asp Leu Asn Gly Ile Phe Tyr Pro Gln Glu Pro
260                     265             270

Leu Phe Gln Thr Gly Phe Asp Gln Gly Gln Glu Asn Trp Ser Asn Gln
        275             280             285

Thr Pro Val Asn Val Gln Val Asp Ser Ile Gly Glu Val Lys Asp His
    290             295             300

Gly Val Leu Gln Thr Leu Glu Gly Ser Thr Asn Pro Val Val Asp Gly
305             310             315             320

Phe Ala Asn Gln Ile Asn Gln Ile Tyr Val Gly Thr Ala Ser Gly Glu
            325             330             335

Pro Ser Gln Ser Glu Ser Ser Asn Ala Ala Pro Val Ile Ser Ser Gly
        340             345             350

Leu Asp Thr Cys Glu Gly Pro Ile Asn Ser Asn Gln Thr Leu Cys Glu
        355             360             365

Ser Ser Trp Lys Gln Val Gly Val Ser Lys Ser Ser Glu Asn Thr Gln
    370             375             380

Asn Arg Val Lys Gly Phe Arg Glu Gln Ile Met Asp Asn Leu Lys Asp
385             390             395             400

Asp Lys Lys Arg Lys Ser Leu Glu Arg Tyr Gly Ser Ile Thr Ser Ala
            405             410             415

Val Ser Asp Asp Lys Gly Asp Thr Thr Lys Lys Arg Arg Val Glu Arg
        420             425             430

Ser Arg Lys Met Ala Glu Ala Lys Glu Arg Asn Ser Thr Pro Ser Val
    435             440             445

Pro Ser Asp Met Gln Ala Val Leu Lys Arg Cys Glu Asn Leu Glu Lys
    450             455             460

Glu Val Arg Ser Leu Lys Leu Asn Leu Ser Phe Met Asn Arg Lys Asp
465             470             475             480

Ser Glu Gln Thr Lys Gln Ile Glu Asp Leu Gln Lys Gln Asn Glu Glu

```
                485                     490                     495


Leu Ala Asp Glu Lys Glu Arg Leu Leu Glu Glu Ile Glu Arg Ile Leu
        500                     505                     510


Ser Glu Thr Glu Lys Met
            515


<210>  3
<211>  1557
<212>  DNA
<213>  Lotus japonicus


<220>
<221>  CDS
<222>  (1)..(1554)
<223>  S50D, S154D


<400>  3
atg gaa ggg agg ggg ttt tct ggt tta tat aga aac tca agt gaa gaa      48
Met Glu Gly Arg Gly Phe Ser Gly Leu Tyr Arg Asn Ser Ser Glu Glu
1               5                  10                  15


ttg ttc ctg aag aca gtg atg gag agc cct att ggt atg cca gtt cct      96
Leu Phe Leu Lys Thr Val Met Glu Ser Pro Ile Gly Met Pro Val Pro
            20                  25                  30


tca atg gag atg ctg ggt ttc aag aat gtt tct caa ggc ttt cgc gca     144
Ser Met Glu Met Leu Gly Phe Lys Asn Val Ser Gln Gly Phe Arg Ala
        35                  40                  45


gat gac gag gag ctt ttc aaa cgc tgg cta aca aat gga gag gga tac     192
Asp Asp Glu Glu Leu Phe Lys Arg Trp Leu Thr Asn Gly Glu Gly Tyr
    50                  55                  60


aat tca tca agc ata ggg ttt agc agt cga tta tca aag agg ata tcc     240
Asn Ser Ser Ser Ile Gly Phe Ser Ser Arg Leu Ser Lys Arg Ile Ser
65                  70                  75                  80


act gaa cta gtt aat gga tct aat cag cta caa gtt ggt gta gcc tca     288
Thr Glu Leu Val Asn Gly Ser Asn Gln Leu Gln Val Gly Val Ala Ser
                85                  90                  95


gat gga aga aac aat gac aaa cca ttc ata caa aat aac ctt ttg gca     336
Asp Gly Arg Asn Asn Asp Lys Pro Phe Ile Gln Asn Asn Leu Leu Ala
            100                 105                 110


aat gat gtt tca ggt gat ttc aat ttt cca atc aga gat cct gtt gat     384
Asn Asp Val Ser Gly Asp Phe Asn Phe Pro Ile Arg Asp Pro Val Asp
        115                 120                 125


aga gaa ctg caa cct agt aac ttg ttt cta gcc aag gcc tgg ttt ctc     432
Arg Glu Leu Gln Pro Ser Asn Leu Phe Leu Ala Lys Ala Trp Phe Leu
```

```
                 130                    135                    140

agt gat caa cga atg aca aga agc cgg gac tct gaa ttg cgg cgg cga    480
Ser Asp Gln Arg Met Thr Arg Ser Arg Asp Ser Glu Leu Arg Arg Arg
145                 150                 155                 160

tat tct gaa atg caa aat ggt cta gcc aca caa gga ata gaa tcc att    528
Tyr Ser Glu Met Gln Asn Gly Leu Ala Thr Gln Gly Ile Glu Ser Ile
                    165                 170                 175

tgc atg gat cct cag cat ggt gct gag gca aca aaa caa gaa gtt gca    576
Cys Met Asp Pro Gln His Gly Ala Glu Ala Thr Lys Gln Glu Val Ala
                180                 185                 190

aat ttc aat ggt tac aat tat ctc tct atg tgt gag ctt cca agt caa    624
Asn Phe Asn Gly Tyr Asn Tyr Leu Ser Met Cys Glu Leu Pro Ser Gln
            195                 200                 205

aag ggt tca ttc atg tct ccg tcc aac tca tgt tca tct aac ttc aac    672
Lys Gly Ser Phe Met Ser Pro Ser Asn Ser Cys Ser Ser Asn Phe Asn
            210                 215                 220

aca cct caa ttt ggc gac atg gat aaa gtt tca tct tgt gta agt atg    720
Thr Pro Gln Phe Gly Asp Met Asp Lys Val Ser Ser Cys Val Ser Met
225                 230                 235                 240

ctg aaa ggg aca tta caa cgc cgg aga ctc agc agt caa ctt gag aaa    768
Leu Lys Gly Thr Leu Gln Arg Arg Arg Leu Ser Ser Gln Leu Glu Lys
                245                 250                 255

gaa gct gca gaa gat gac tta aat gga att ttt tat cct caa gaa cct    816
Glu Ala Ala Glu Asp Asp Leu Asn Gly Ile Phe Tyr Pro Gln Glu Pro
                260                 265                 270

ctt ttc caa act ggc ttt gat caa gga caa gaa aac tgg agt aat caa    864
Leu Phe Gln Thr Gly Phe Asp Gln Gly Gln Glu Asn Trp Ser Asn Gln
                275                 280                 285

acg cca gta aat gtt caa gta gac tct att ggt gaa gtt aag gat cat    912
Thr Pro Val Asn Val Gln Val Asp Ser Ile Gly Glu Val Lys Asp His
                290                 295                 300

gga gtc ctg caa aca cta gaa gga tcc aca aac cct gtc gtt gat ggt    960
Gly Val Leu Gln Thr Leu Glu Gly Ser Thr Asn Pro Val Val Asp Gly
305                 310                 315                 320

ttt gca aat cag ata aac caa atc tat gtc gga aca gct tct gga gaa   1008
Phe Ala Asn Gln Ile Asn Gln Ile Tyr Val Gly Thr Ala Ser Gly Glu
                325                 330                 335

cct tct caa agt gaa tcc tct aat gct gca cca gta atc tcc tct ggt   1056
Pro Ser Gln Ser Glu Ser Ser Asn Ala Ala Pro Val Ile Ser Ser Gly
                340                 345                 350

tta gac aca tgc gaa ggt ccc ata aac tcg aat caa act ctc tgc gaa   1104
Leu Asp Thr Cys Glu Gly Pro Ile Asn Ser Asn Gln Thr Leu Cys Glu
                355                 360                 365
```

```
agc tca tgg aaa caa gta gga gtg agt aaa agt tca gaa aat act caa      1152
Ser Ser Trp Lys Gln Val Gly Val Ser Lys Ser Ser Glu Asn Thr Gln
    370             375             380

aat aga gtc aaa ggt ttc aga gaa cag atc atg gat aat ctg aaa gat      1200
Asn Arg Val Lys Gly Phe Arg Glu Gln Ile Met Asp Asn Leu Lys Asp
385             390             395             400

gat aag aag aga aaa agt cta gaa aga tat gga tct ata aca tca gct      1248
Asp Lys Lys Arg Lys Ser Leu Glu Arg Tyr Gly Ser Ile Thr Ser Ala
            405             410             415

gtt tca gat gac aag gga gac acc act aaa aag cgt agg gtg gaa cgc      1296
Val Ser Asp Asp Lys Gly Asp Thr Thr Lys Lys Arg Arg Val Glu Arg
            420             425             430

tca agg aaa atg gct gaa gct aag gaa aga aat tcg aca cca tca gtt      1344
Ser Arg Lys Met Ala Glu Ala Lys Glu Arg Asn Ser Thr Pro Ser Val
            435             440             445

ccc tca gat atg caa gct gtc ttg aag cgg tgc gaa aac ctt gag aag      1392
Pro Ser Asp Met Gln Ala Val Leu Lys Arg Cys Glu Asn Leu Glu Lys
            450             455             460

gaa gtt cga tcg cta aaa ctc aac ttg tcc ttc atg aat aga aag gat      1440
Glu Val Arg Ser Leu Lys Leu Asn Leu Ser Phe Met Asn Arg Lys Asp
465             470             475             480

tct gaa caa aca aag cag ata gaa gac ctt cag aag cag aat gaa gag      1488
Ser Glu Gln Thr Lys Gln Ile Glu Asp Leu Gln Lys Gln Asn Glu Glu
            485             490             495

ctg gca gat gaa aaa gag cgc ctc ctc gaa gag att gaa aga att cta      1536
Leu Ala Asp Glu Lys Glu Arg Leu Leu Glu Glu Ile Glu Arg Ile Leu
            500             505             510

tca gaa act gaa aaa atg taa                                          1557
Ser Glu Thr Glu Lys Met
            515
```

```
<210>   4
<211>   518
<212>   PRT
<213>   Lotus japonicus

<400>   4
```

```
Met Glu Gly Arg Gly Phe Ser Gly Leu Tyr Arg Asn Ser Ser Glu Glu
1               5               10              15
```

```
Leu Phe Leu Lys Thr Val Met Glu Ser Pro Ile Gly Met Pro Val Pro
            20              25              30
```

```
Ser Met Glu Met Leu Gly Phe Lys Asn Val Ser Gln Gly Phe Arg Ala
        35                  40                  45

Asp Asp Glu Glu Leu Phe Lys Arg Trp Leu Thr Asn Gly Glu Gly Tyr
        50                  55                  60

Asn Ser Ser Ser Ile Gly Phe Ser Ser Arg Leu Ser Lys Arg Ile Ser
65                  70                  75                  80

Thr Glu Leu Val Asn Gly Ser Asn Gln Leu Gln Val Gly Val Ala Ser
                85                  90                  95

Asp Gly Arg Asn Asn Asp Lys Pro Phe Ile Gln Asn Asn Leu Leu Ala
            100                 105                 110

Asn Asp Val Ser Gly Asp Phe Asn Phe Pro Ile Arg Asp Pro Val Asp
            115                 120                 125

Arg Glu Leu Gln Pro Ser Asn Leu Phe Leu Ala Lys Ala Trp Phe Leu
        130                 135                 140

Ser Asp Gln Arg Met Thr Arg Ser Arg Asp Ser Glu Leu Arg Arg Arg
145                 150                 155                 160

Tyr Ser Glu Met Gln Asn Gly Leu Ala Thr Gln Gly Ile Glu Ser Ile
                165                 170                 175

Cys Met Asp Pro Gln His Gly Ala Glu Ala Thr Lys Gln Glu Val Ala
            180                 185                 190

Asn Phe Asn Gly Tyr Asn Tyr Leu Ser Met Cys Glu Leu Pro Ser Gln
            195                 200                 205

Lys Gly Ser Phe Met Ser Pro Ser Asn Ser Cys Ser Ser Asn Phe Asn
        210                 215                 220

Thr Pro Gln Phe Gly Asp Met Asp Lys Val Ser Ser Cys Val Ser Met
225                 230                 235                 240

Leu Lys Gly Thr Leu Gln Arg Arg Arg Leu Ser Ser Gln Leu Glu Lys
                245                 250                 255

Glu Ala Ala Glu Asp Asp Leu Asn Gly Ile Phe Tyr Pro Gln Glu Pro
```

260                    265                    270

Leu Phe Gln Thr Gly Phe Asp Gln Gly Gln Glu Asn Trp Ser Asn Gln
        275                280                285

Thr Pro Val Asn Val Gln Val Asp Ser Ile Gly Glu Val Lys Asp His
        290                295                300

Gly Val Leu Gln Thr Leu Glu Gly Ser Thr Asn Pro Val Val Asp Gly
305                310                315                320

Phe Ala Asn Gln Ile Asn Gln Ile Tyr Val Gly Thr Ala Ser Gly Glu
            325                330                335

Pro Ser Gln Ser Glu Ser Ser Asn Ala Ala Pro Val Ile Ser Ser Gly
        340                345                350

Leu Asp Thr Cys Glu Gly Pro Ile Asn Ser Asn Gln Thr Leu Cys Glu
        355                360                365

Ser Ser Trp Lys Gln Val Gly Val Ser Lys Ser Ser Glu Asn Thr Gln
    370                375                380

Asn Arg Val Lys Gly Phe Arg Glu Gln Ile Met Asp Asn Leu Lys Asp
385                390                395                400

Asp Lys Lys Arg Lys Ser Leu Glu Arg Tyr Gly Ser Ile Thr Ser Ala
            405                410                415

Val Ser Asp Asp Lys Gly Asp Thr Thr Lys Lys Arg Arg Val Glu Arg
        420                425                430

Ser Arg Lys Met Ala Glu Ala Lys Glu Arg Asn Ser Thr Pro Ser Val
        435                440                445

Pro Ser Asp Met Gln Ala Val Leu Lys Arg Cys Glu Asn Leu Glu Lys
        450                455                460

Glu Val Arg Ser Leu Lys Leu Asn Leu Ser Phe Met Asn Arg Lys Asp
465                470                475                480

Ser Glu Gln Thr Lys Gln Ile Glu Asp Leu Gln Lys Gln Asn Glu Glu
            485                490                495

Leu Ala Asp Glu Lys Glu Arg Leu Leu Glu Glu Ile Glu Arg Ile Leu
            500                 505                 510

Ser Glu Thr Glu Lys Met
        515

<210> 5
<211> 19
<212> DNA
<213> artificial

<220>
<223> primer for the amplification of the L. japonicus CYCLOPS gene

<400> 5
caccatggaa gggaggggg                                              19

<210> 6
<211> 29
<212> DNA
<213> artificial

<220>
<223> primer for the amplification of the L. japonicus CYCLOPS gene

<400> 6
ttacattttt tcagtttctg atagaattc                                  29

<210> 7
<211> 27
<212> DNA
<213> artificial

<220>
<223> primer for the amplification of the L. japonicus gene promoter

<400> 7
caccccaaac tatcaggtca agtctgc                                     27

<210> 8
<211> 32
<212> DNA
<213> artificial

<220>
<223> primer for the amplification of the L. japonicus gene promoter

<400> 8
tcaaagtcga cgtttggctc aacagcactt tc                               32

```
<210>  9
<211>  28
<212>  DNA
<213>  artificial

<220>
<223>  primer for the introduction of the S50D mutation in the L.
       japonicus CYCLOPS gene

<400>  9
ctttcgcgca gatgacgagg agcttttc                                         28


<210>  10
<211>  28
<212>  DNA
<213>  artificial

<220>
<223>  primer for the introduction of the S50D mutation in the L.
       japonicus CYCLOPS gene

<400>  10
gaaaagctcc tcgtcatctg cgcgaaag                                         28


<210>  11
<211>  31
<212>  DNA
<213>  artificial

<220>
<223>  primer for the introduction of the S154D mutation in the L.
       japonicus CYCLOPS gene

<400>  11
gacaagaagc cgggactctg aattgcggta c                                     31


<210>  12
<211>  31
<212>  DNA
<213>  artificial

<220>
<223>  primer for the introduction of the S154D mutation in the L.
       japonicus CYCLOPS gene

<400>  12
gtaccgcaat tcagagtccc ggcttcttgt c                                     31
```

**Description**

The present invention provides a plant comprising a nucleotide sequence of a (mutant) CYCLOPS gene, wherein said nucleotide sequence comprises at a first position corresponding to position 148-150 of the nucleotide sequence shown in SEQ ID No:1 and/or at a second position corresponding to position 460-462 of the nucleotide sequence shown in SEQ ID No:1 a codon encoding an amino acid mimicking the effects of phosphorylation. Also, plants are provided which comprise a CYCLOPS protein having at a first position corresponding to position 50 of the amino acid sequence of the CYCLOPS protein shown in SEQ ID No:2 and/or at a second position corresponding to position 154 of the amino acid sequence of the CYCLOPS protein shown in SEQ ID No:2 an amino acid mimicking the effects of phosphorylation. The plants of the present invention are capable of developing root nodules, preferably spontaneously developing nodules, preferably in the absence of rhizobia and/or rhizobial signal molecules.

Plants benefit from interactions with symbiotic bacteria and fungi, to improve their phosphate and nitrogen supply, and it is those interactions in particular that hold the promise of becoming an integral part of a more sustainable agriculture.

Probably the most widespread strategy of plants to improve the uptake of phosphate and other minerals is to form symbiosis with arbuscular mycorrhiza (AM) fungi.

Similarly, one of the most efficient nitrogen-fixing symbioses is the root nodule symbiosis (RNS) of legumes with rhizobia.

The growth of agricultural crops is generally limited by the availability of nitrogen, and at least 50% of global requirement is met by the application of synthetic fertilisers in the form of ammonia, nitrate or urea. However, there is a growing need to exploit one of the most important natural sources of nitrogen for agriculture, namely biological nitrogen fixation.

The primary source of biological nitrogen fixation are Gram negative rhizobia and the Gram positive actinobacterium *Frankia* spp, which are a small group of prokaryotes that produce nitrogenases, and form endosymbiotic associations with plants conferring the ability to fix nitrogen. Although many plants can associate with nitrogen-fixing bacteria, only a few plants form endosymbiotic associations with rhizobia or *Frankia,* which are unique in that most of the nitrogen is transferred to and assimilated by the host plant. The Leguminosae plant family, which includes soybean, bean, pea, peanut, chickpea, cowpea, lentil, pigeonpea, alfalfa and clover, are the most agronomically important members of this small group of nitrogen-fixing plants. Biological nitrogen fixation via the endosymbiotic association reduces the need for expensive nitrogen fertilizers in legume crops and is an important feature of sustainable agriculture. Legumes can also utilize nitrogen available in the soil, such that when levels of soil nitrate are high, nodule formation is suppressed and the plant shifts from nitrogen metabolism to growth on nitrate.

In particular, legume plants can establish endosymbiotic interactions with nitrogen-fixing rhizobia and phosphate-delivering arbuscular mycorrhiza (AM) fungi. In the initial stages of the root nodule symbiosis (RNS), legume root hairs form a tight curl in which rhizobia are entrapped. From this closed infection pocket, the bacteria are guided by plant membrane-delimited infection threads (ITs) into the root nodule, a specialized organ developed by the plant to provide an optimized environment for nitrogen fixation. AM fungal hyphae are guided through epidermal and cortical cells toward the inner cortex, where arbuscules, highly branched intracellular symbiotic structures, are formed. Intracellular infection by rhizobia and AM fungi is preceded by an exchange of specific signaling molecules. Rhizobia produce lipochito-oligosaccharides (Nod factors) that activate host plant responses including root hair deformation, and preinfection thread formation, which are structures that determine the path of IT growth through the root, and initiation of cortical cell division. One of the earliest plant responses to stimulation by Nod factors is calcium-spiking, which consists of perinuclear oscillations of calcium concentration in root cells.

Thus, rhizobium-legume symbiosis involves the interaction of a set of plant and bacterial genes in a complex process leading to the initiation and development of root nodules. Organogenesis of nodules is triggered by the rhizobial microsymbiont, but the legume host plant encodes the developmental program responsible for building the nodule tissues and for regulating the process. Lipo-chitin-oligosaccharides (Nod-factors) synthesized and secreted by Rhizobia are major signal molecules that trigger the process of organogenesis including nodule formation. The major Nod-factor secreted by the Mesorhizobium loti microsymbiont of Lotus is a pentameric N-acetylglucosamine carrying a cis-vaccenic acid and a carbamoyl group at the non-reducing terminal residue together with a 4-O-acetylfucose at the reducing terminal residue. Perception of Nod-factor in Lotus is mediated by NFR1 and NFR5 receptor kinases (Radutoiu et al. (2003), Nature 425, 585-592; Madsen et al. (2003), Nature 425, 637-640), that together with an LRR receptor-kinase gene, SymRK, communicate with a common signal transduction pathway, shared with mycorrhizal symbiosis (Oldroyd and Downie, 2004 Nature Reviews 5: 566- 576). This symbiosis pathway common to AM and RNS is encoded by seven genes, *SYMRK, CASTOR, POLLUX, NUP85, Nup133, NENA, CCaMK* and *CYCLOPS.* These 'common symbiosis genes' genes are conserved in angiosperms consistent with the ancient nature and widespread occurrence of AM, which is formed by 70-80% of all land plant species

Analysis of mutants has shown that NFR1/NFR5 receptor(s), SymRK encoded LRR protein kinase, CASTOR/POLLUX cation channel(s), nucleoporins SEH1 (Groth et al. (2010), Plant Cell 22, 2509-2526), NUP85 and NUP133 are required for the induction of calcium spiking, one of the earliest physiological responses detectable in root hairs exposed to purified Nod-factor.

Specifically, the predicted protein products of these 'common symbiosis genes' include a receptor kinase that is also required for actinorhiza symbiosis and has been implicated in the evolution of nodulation (Markmann et al. (2008), PLoS Biol. 6, e68). Together with nuclear envelope localized ion channels (Charpentier et al. (2009), Plant Cell 20, 3467-3479) and components of the NUP84 sub-complex of the nuclear pore (Kanamori et al. (2006), Proc Natl Acad Sci USA 103, 359-364; Saito et al. (2007); Plant Cell; Groth et al., (2010), cited above), these components act upstream of symbiosis induced calcium spiking, which is likely to be decoded by a complex formed by a calcium- and calmodulin-dependent protein kinase, CCaMK (Tirichine et al. (2006), Nature 441, 1153-1156) and CYCLOPS, a nuclear protein with a coiled-coil domain (Yano et al. (2008), Proc. Natl. Acad. Sci. USA 105: 20540-20545).

CCaMK is a calcium and calmodulin dependent protein kinase that is believed to decode calcium-spiking signatures that are induced within minutes upon symbiotic stimulation of root cells (Tirichine et al., (2006), cited herein). CCaMK interacts with and phosphorylates CYCLOPS (Yano et al., 2008, cited herein).
In particular, CCaMK forms a preassembled complex with CYCLOPS in the nucleus, as interaction occurs in the absence of a calcium stimulus. cyclops mutants are defective AM and RNS, but show a wildtype-like calcium spiking response (Yano et al., (2008), cited herein). The mutant name is derived from its particular phenotype characterized by a root hair curl entrapping a bacterial microcolony. At this early stage, progression of infection via an infection thread is blocked. Sequence analysis of CYCLOPS predicts a protein of unknown function with two nuclear localization signals and a short coiled-coil at the C-terminus. The observation that spontaneous nodules are formed in cyclops mutants transformed with gain-of-function CCaMK, suggests a bifurcation of signal transduction at, or below CCaMK. According to this model, CCaMK mediates microbial infection via CYCLOPS in the epidermis, while nodule organogenesis is initiated via yet unidentified CCaMK targets in the cortex.
In addition, CYCLOPS was shown to interact with CCaMK both in yeast in the Gal4 yeast two-hybrid (Y2H) assay and in planta in Bimolecular Fluorescence Complementation (BiFC) experiments. BIFC is a non-invasive fluorescent-based technique that allows detection of protein-protein interactions in living cells, and furthermore can be used to determine subcellular localization of the interacting proteins, and if it changes over time, without requiring addition of external agents. BiFC is based upon reconstitution of split non-fluorescent GFP variants, primarily YFP, to form a fluorescent fluorophore. Accordingly, it was shown that CCaMK and CYCLOPS interact in the nucleus of plant cells where they presumably form a pre-assembled complex as interaction is detected in the absence of a calcium stimulus. In vitro kinase assays identified CYCLOPS as phosphorylation substrate of CCaMK.

To this end, the cyclops mutant phenotype indicates that CYCLOPS is located downstream of CCaMK, as in contrast to cyclops, ccamk mutants do not show a root hair curling response towards rhizobia. Further, upon rhizobial infection, cyclops mutants form small, uninfected nodule primordia, while ccamk loss-of-function mutants do not initiate nodule organogenesis. In addition, on the basis of the observation that a constitutively active derivative of CCaMK could initiate nodule formation in a cyclops mutant, it was concluded that CYCLOPS is dispensable for nodule organogenesis (Yano et al. (2008), cited herein).

In sum, the identification of the key genes, which encode functions which are indispensable for establishing a nitrogen fixing system in legumes, and their transfer and expression in non-nodulating plants, has long been a goal of molecular plant breeders. This could have a significant agronomic impact on the cultivation of cereals such as rice, where production of two harvests a year may require fertilisation with up to 400 kg nitrogen per hectare.

Hence, there is a need to transfer the nodule formation capability and nitrogen fixation properties of legume crops into non-nodulating crops in order to meet the nutritional needs of a growing global population, while minimising the future use of nitrogen fertilisers and their associated negative environmental impact.

Hence, the technical problem of the present invention is to comply with this need.

The present invention addresses this need and thus provides as a solution to the technical problem embodiments pertaining to plants, in particular to land plants capable of symbiosis with arbuscular mycorrhiza such as non-legume plants, that are capable of spontaneous organogenesis, in particular capable of spontaneous root nodule formation, because of the activity of a CYCLOPS protein having (an) amino acid(s) that mimic phosphorylation of (normally) in planta phosphorylated amino acids, thereby providing the structures (root nodules) required by rhizobia to become beneficial for plants in that they fix nitrogen, as well as methods of uses of these plants. These embodiments are characterized and described herein, shown in the Figures, illustrated in the Examples, and reflected in the claims.

It must be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.
All publications and patents cited in this disclosure are incorporated by reference in their entirety. To the extent the material incorporated by reference contradicts or is inconsistent with this specification, the specification will supersede any such material.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.

In each instance herein any of the terms "comprising", "consisting essentially of" and "consisting of" may be replaced with either of the other two terms.

Several documents are cited throughout the text of this specification. Each of the documents cited herein (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention.

In a first aspect the present invention provides a plant comprising a nucleotide sequence of a (mutant) CYCLOPS gene, wherein said nucleotide sequence comprises at a first position corresponding to position 148-150 of the (wild-type) nucleotide sequence shown in SEQ ID No:1 and/or at a second position corresponding to position 460-462 of the nucleotide sequence shown in SEQ ID No:1 a codon encoding an amino acid mimicking the effects of phosphorylation.

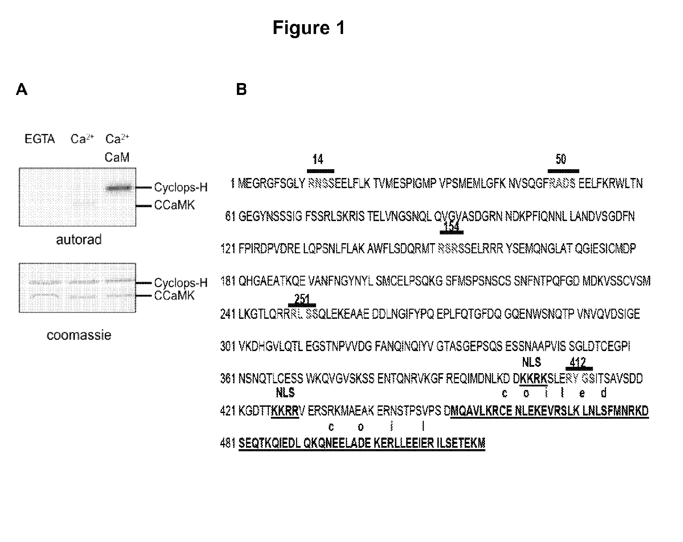

As mentioned above, experimentally demonstrated and shown in Figure 1, CYCLOPS contains in total 5 phosphorylation sites. Accordingly, it is a preferred embodiment of the present invention that a nucleotide sequence comprising at a first position corresponding to position 148-150 of the (wild-type) nucleotide sequence shown in SEQ ID No:1 and/or at a second position corresponding to position 460-462 of the nucleotide sequence shown in SEQ ID No:1, comprises in addition at a further position corresponding to position 40-42 and/or 751-753 and/or 1234-1236 of the nucleotide sequence shown in SEQ ID No:1, a codon encoding an amino acid mimicking the effects of phosphorylation.

Similarly, preferably a nucleotide sequence comprising at one or more positions corresponding to position(s) 40-42, 148-150, 460-462, 751-753 and/or 1234-1236 of the nucleotide sequence shown in SEQ ID No:1 a codon encoding an amino acid mimicking the effects of phosphorylation, is envisaged that shows the same effect (activity) as the CYCLOPS double D mutant. Said activity can be tested as described elsewhere herein.

Likewise, the present invention provides a plant encoding a (mutant) CYCLOPS protein having at a first position corresponding to position 50 of the amino acid sequence of the (wild-type) CYCLOPS protein shown in SEQ ID No:2 and/or at a second position corresponding to position 154 of the amino acid sequence of the CYCLOPS protein shown in SEQ ID No:2 an amino acid mimicking the effects of phosphorylation.

Accordingly, it is a preferred embodiment of the present invention that a (mutant) CYCLOPS protein comprises at a first position corresponding to position 50 of the amino acid sequence shown in SEQ ID No:2 and/or at a second position corresponding to position 154 of the amino acid sequence shown in SEQ ID No:2, comprises in addition at a further position corresponding to position 14 and/or 251 and/or 412 of the amino acid sequence shown in SEQ ID No: 2, a an amino acid mimicking the effects of phosphorylation.

Similarly, preferably a (mutant) CYCLOPS protein comprising at one or more positions corresponding to position(s) 14, 50, 154, 251 and/or 412 of the amino acid sequence shown in SEQ ID No:2 an amino acid mimicking the effects of phosphorylation, is envisaged that shows the same effect (activity) as the CYCLOPS double D mutant. Said activity can be tested as described elsewhere herein.

In order to understand the molecular mechanisms involved in the decoding and transduction of the respective symbiosis-specific calcium oscillations, the present inventors aimed at a detailed structural and functional characterization of the nuclear CCaMK/CYCLOPS signaling complex by means of genetical, physiological and biochemical approaches. In this context the present inventors were also addressing the intriguing specificity and underlying mechanisms leading to distinct responses (e.g. nodule formation in RNS but not during AM) downstream of calcium spiking.

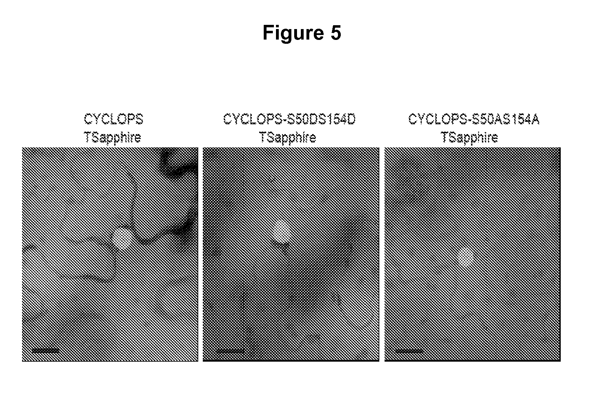

Most surprisingly, in their detailed analysis of *CYCLOPS* mutants, the present inventors observed that transformation of a *CYCLOPS* gene, which was modified so as to encode at phosphorylation sites (i.e., at a serine residues which can be phosohorylated) an acidic amino acid such as aspartate or glutamate, into plants which even had an inactive CCaMK, leads to spontaneous organogenesis, in particular to root nodule formation. Thus, though it was previously reported by Yano et al. (cited herein) that CYCLOPS is dispensable for organogenesis, in particular for nodule formation, the opposite holds true. Similarly, Hayashi et al. (2010), Plant J. 63, 141-154 points out that CYCLOPS is not involved in nodule organogenesis (see page 148, legend of Figure 5). Indeed, it was previously observed that a constitutively active CCaMK complements a *cyclops* mutant plant insofar that nodules were formed, it has been found that CYCLOPS plays an important role. Specifically, "activated", i.e., CYCLOPS having at one or one or more (preferably at two) phosphorylation sites an acidic amino acid (such as "CYCLOPS double D" having an aspartate at a position corresponding to position 50 and 154, respectively, of the amino acid sequence shown in SE ID No:2) rather than a serine residue is capable of forming nodules.

This result is of outstanding interest, because it does not only demonstrate that CYCLOPS is positioned upstream of the entire organogenesis program, but also that it can be engineered to become solosufficient for triggering *de novo* meristem and organ formation. In sum, by introducing phosphomimetic amino acids at amino acids which are phosphorylated *in planta* i.e., replacing amino acids which are subject to phosphorylation by acidic amino acids; in particular a serine residue of a CYCLOPS protein at a position corresponding to position 50 and/or 154 of the amino aid sequence of SEQ ID No:2), it seems possible to induce nodules - even in non-legume crop plants. If these nodules are colonized by bacteria and/or fungi as symbionts, plants could tremendously benefit and less or preferably even no fertilizer may be required in agriculture.

In sum, the present inventors' finding may prove instrumental in biotechnological approaches with the aim to provide cultivars with improved nutrition through genetically optimised symbiosis. Since the common symbiosis genes including *CYCLOPS* are widespread in angiosperms including major fodder, fruit, vegetable and cereal crops, *CYCLOPS* mutant alleles encoding CYCLOPS mimicking *in planta* phosphorylation (such as the double D mutant) may be one component in a multistep strategy to transfer the root nodule symbiosis to non-legume crop plants.

In their work subsequent to the Yano et al. publication (cited herein), the present inventors have used different purification and assay conditions and could consistently observe that full length CYCLOPS is phosphorylated by CCaMK in the presence of calcium/CaM (Figure 1A). By mass spectrometric analysis of phosphorylated CYCLOPS, five *in vitro* phosphorylation sites were identified. The sites are conserved in CYCLOPS orthologs from legumes and other AM forming angiosperms and are located in the sequence motif RXXS, implying this motif to be a substrate recognition site of CCaMK (Figure 1 B). To test the relevance of the *in vitro* phosphorylation sites for symbiosis establishment, they generated single-site and multiple-site, unphosphorylateable as well as phosphomimetic versions of CYCLOPS and analyzed the symbiotic phenotype *in planta.* Of note, though the present inventors used genomic *L. japonicus* DNA (containing exons and introns) for their experiments, it is apparent that cDNA or any other DNA as described herein can also be used.

More specifically, the five identified phosphorylated serine residues were replaced by alanine by site-directed mutagenesis, in order to create un-phosphorylateable residues. The resulting ,,Quintupie-A" genomic mutant construct equipped with the *CYCLOPS* promoter and fused to an N-terminal 3xHA-tag was introduced into the *cyclops-3* mutant background by hairy root transformation and complementation of AM and RNS was analyzed five weeks post infection (wpi). When compared to the wildtype control, the ,,Quintuple-A mutant" did not complement RNS and AM.

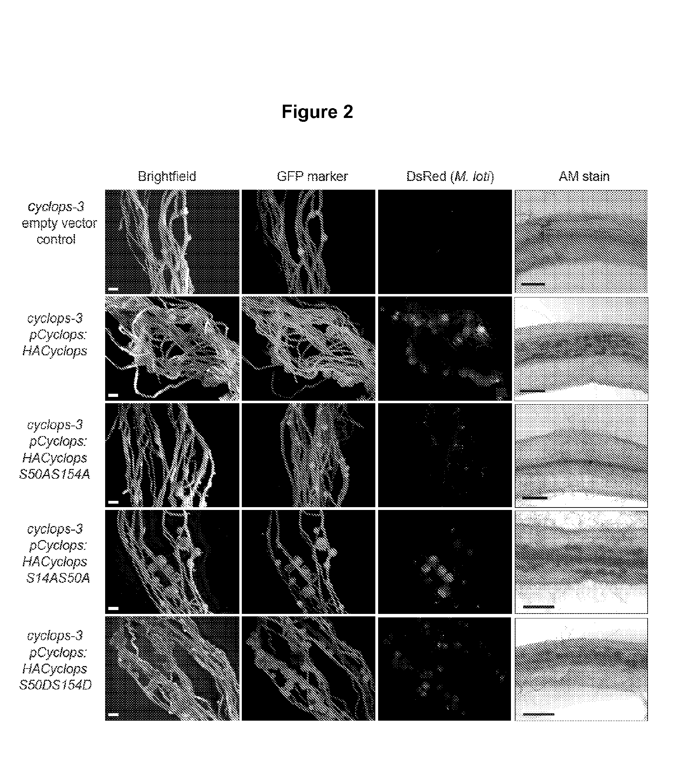

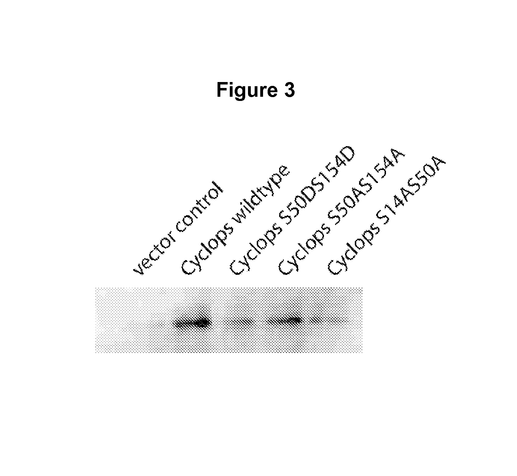

Consequently, the five single site mutants and combinations thereof were tested for symbiosis complementation, and successfull expression of the tagged proteins was confirmed by protein blot analysis (Figure 3). While all single site mutants complemented both types of symbioses, the simultaneous mutation of S50A and S154A was sufficient to abolish symbiosis establishment (Figure 2). In contrast simultaneous replacement of S14A and S50A, or S14A and S154A allowed infection by the AM fungus and *M. loti* (Figure 2 and data not shown for CYCLOPS-S14AS154A), suggesting that lack of complementation by CYCLOPS-S50AS154A was specifically due to impairment of phosphorylation.

CYCLOPS S50 and S154 orthologous phosphorylation sites of I̲nteracting P̲rotein of D̲MI3 (IPD3, the Medicago orthologue of *Lotus* CYCLOPS (Messinese et al. (2007). Mol Plant Microbe Interact. 8, 912-921) were independently identified in *Medicago* roots (Grimsrud et al. (2010), Plant Physiol. 152, 19-28), thus confirming these sites to be phosphorylated *in vivo.* However, Grimsrud et al., do not describe anything that goes beyond the mere showing of phosphorylated IPD3.

Further, five single site phosphomimetic serine to aspartate replacement constructs and the *pUB:CYCLOPS-S50DS154D* double mutant construct were analyzed for symbiotic performance in the *cyclops-3* mutant background. In this approach, all constructs rescued both symbioses (Figure 5). In summary, the *in vivo* phosphorylation sites S50 and S154 could be pinpointed as essential and redundant for symbiosis signaling since the phosphomimetic CYCLOPS-S50DS154D and the unphosphorylateable S50AS154A replacements were compatible and incompatible with symbiosis, respectively while the single site mutants were not affected.

Among these constructs, one construct expressing a protein carrying at the same time S50D and S154D replacements (sometimes referred herein as "CYCLOPS double D") resulted surprisingly in the production of spontaneous nodules *in the absence* of rhizobia.

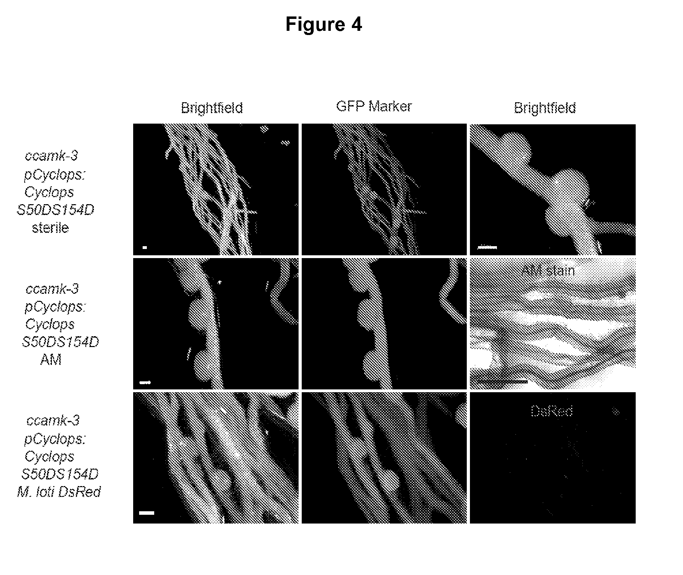

In detail, the *pUB:CYCLOPS-S50DS154D* construct was introduced into the *ccamk-3* background and the symbiosis phenotype of non-inoculated plants, plants inoculated with AM fungi, or inoculated with M. *loti* was assessed 5 weeks post infection (wpi). The construct failed to restore infection with AM fungi or M. *loti* (Figure 4). Most surprisingly, transformation of *CYCLOPS-S50DS154D* was sufficient for the spontaneous formation of nodules in the absence of rhizobia (Figure 4), demonstrating that CYCLOPS double D is upstream of the organogenesis program.

Thus, though it was previously reported in the Yano et al. publication (cited herein) and confiremd by Hayashi et al.

(cited herein) that CYCLOPS is dispensable for nodule formation/organogenesis, the present inventors have observed the opposite.

In order to determine the location of the double D CYCLOPS mutant as well as that of the wild type, C-terminal fusions of CYCLOPS wildtype, CYCLOPS-S50AS154A and CYCLOPS-S50DS154D to the fluorophore T-Sapphire were tested for their subcellular localization in N. *benthamiana* leaf epidermis cells. In all cases the proteins localized to the nucleus (Figure 5). This result shows that the CYCLOPS double D mutant does not behave different than wildtype and, thus, there is no dislocalization which could have been responsible for the observed effect in nodule formation.

Hence, mutating CYCLOPS may pave the way to induce nodules - even in non-legume crop plants. If these nodules are colonized by nitrogen-fixing bacteria as symbionts, plants could tremendously benefit and less or even no fertilizer may be required in agriculture.

Therefore, the present invention may prove instrumental in biotechnological approaches with the aim to provide cultivars with improved nutrition through genetically optimised symbiosis. Since the common symbiosis genes including *CYCLOPS* are widespread in angiosperms including major cereal crops, for example, CYCLOPS double D may be one component in a multistep strategy to transfer the root nodule symbiosis to non-legume crop plants.

As used herein unless clearly indicated otherwise, the term "plant" intended to mean a plant at any developmental stage, as well as any part or parts of a plant that may be attached to or separate from a whole intact plant. Such parts of a plant include, but are not limited to, organs, tissues, and cells of a plant including, plant calli, plant clumps, plant protoplasts and plant cell tissue cultures from which plants can be regenerated. Examples of particular plant parts include a stem, a leaf, a root, an inflorescence, a flower, a floret, a fruit, a pedicle, a peduncle, a stamen, an anther, a stigma, a style, an ovary, a petal, a sepal, a carpel, a root tip, a root cap, a root hair, a leaf hair, a seed hair, a pollen grain, a microspore, an embryos, an ovule, a cotyledon, a hypocotyl, an epicotyl, xylem, phloem, parenchyma, endosperm, a companion cell, a guard cell, and any other known organs, tissues, and cells of a plant. Furthermore, it is recognized that a seed is a plant.

Plants of the present invention preferably also include plants, preferably land plants which can be infected by symbiotic arbuscular mycorrhiza (AM) fungi. Also included by the term "plant" are dicotyledones, monocotyledons, gymnosperms, angiosperms including plants of agricultural or pharmaceutical value which are capable to form AM), liverworths, mosses, hornworts, lycophytes, monilophytes, gymnosperms and angiosperms, but plants of the present invention are not limited thereto.

Preferably, plants (source material) that are intended to then contain/express a mutant CYCLOPS nucleic acid of the present invention do not have a *cyclops, ccamk, Ihk1* or the like (inactivating) mutation, i.e., are inactive as regards these genes. Preferably, plants (source material) that are intended to then contain/express a mutant CYCLOPS nucleic acid of the present invention do not have a *ccamk* and/or *Ihk1* gain-of-function.

Also preferred are non-legume plants including non-legume crop plants. Further preferred plants include all angiosperm plants with nutritional or pharmaceutical vale that form AM, these include among many thousand other plants, important crops such as rice, barley, wheat, oat, rye, maize, sugar cane, poaceae grasses, cucumber, melon, tomato, wine, etc.

The only limitations may be very few exceptional plant families that have recently lost the ability to form AM, including the Brassicaceae (including rape), the Resedeaceae and the Chenopodiaceae (including sugar beet (Beta vulgaris). But since the mutations responsible for the loss of AM are unknown in most cases, CYCLOPS double D may still work in these plants.

It is known from Wang et al. (2010), New Phytologist 186, 514-525 (and supporting information) that three mycorrhizal genes (orthologs of *DMI1, DMI3* and *IPD3*) are present in the common ancestors of land plants. Wang et al. investigated the evolutionary histories and functional conservation of three genes required for mycorrhiza formation in legumes and rice (Oryza sativa), *DMI1, DM13* and *IPD3.* The genes were isolated from nearly all major plant lineages. Phylogenetic analyses showed that they had been vertically inherited since the origin of land plants. Further, cross-species mutant rescue experiments demonstrated that DM13 genes from liverworts and hornworts could rescue Medicago truncatula *dmi3* mutants for mycorrhiza formation. Yeast two-hybrid assays also showed that bryophyte DMI3 proteins could bind to downstream-acting M. trunculata IPD3 protein. Finally, molecular evolutionary analyses revealed that these genes were under purifying selection for maintenance of their ancestral functions in all mycorrhizal plant lineages.

Thiese findings suggest a key role of mycorrhizas in the colonization of land by plants and also that almost all land plants have the capability of forming symbiotic mycorrhizas. This analysis also shows that the basal genes required for the root nodule symbioses with rhizobial or Frankia bacteria are widespread in the plant kingdom. Specifically, Wang et al. show that diverse plants have genes required for forming symbioses with arbuscular mycorrhizas and/or with rhizobia. More specifically, Wang et al. show that mycorrhizal genes were present in the common ancestor of land plants, and that their functions were largely conserved during land plant evolution. The evidence presented here strongly suggests that plant-mycorrhizal fungus symbiosis was one of the key processes that contributed to the origin of land flora.

Hence, the results presented by Wang et al. allow two conclusions to be drawn. The first conclusion is that orthologous copies of the three mycorrhizal genes were present in the common ancestor of land plants, because they are present in nearly all major lineages of extant land plants, including liverworts - the earliest-diverging lineage of land plants - and that these genes have been vertically inherited during land plant evolution.

Accordingly, given the fact that CYCLOPS belongs to the IPD3 proteins (as described in detail herein elsewhere) and that IPD3 is present in almost all land plants and that further genes required for mycorrhizas are also present in these plants (see Table 1 of Wang et al., also shown herein below), it is more than plausible and reasonable to conclude that plants of the present invention when containing and/or expressing a mutant CYCLOPS gene/protein of the present invention have the capability to spontaneously develop nodules, in particular root nodules, thereby providing the structures required for symbiosis with rhizobial bacteria (and for symbiosis with arbuscular mycorrhiza).

When referred to herein the terms "nucleotide sequence(s)", "polynucleotide(s)", "nucleic acid sequence(s)" "nucleic acid(s)", "nucleic acid molecule" are used interchangeably and refer to nucleotides, either ribonucleotides or deoxyribonucleotides or a combination of both, in a polymeric unbranched form of any length. Nucleic acid sequences include DNA, cDNA, genomic DNA, RNA, synthetic forms and mixed polymers, both sense and antisense strands, or may contain non-natural or derivatized nucleotide bases, as will be readily appreciated by those skilled in the art.

A "gene" when used herein is, so to say, a species of a nucleotide sequence and comprises a coding sequence for a gene (here the CYCLOPS gene) and, optionally a 5'-UTR (containing, for example, expression control elements such as a promoter) and/or 3'-UTR (containing, for example, a termination signal sequence). The gene may be composed of exons and introns or may be free of introns, thus merely composed of exons. It may be composed of DNA, genomic DNA or cDNA.

The term "expressing" and all its grammatical forms thereof means that a nucleic acid encoding a mutant or wild-type CYCLOPS protein is transcribed and translated into protein in a host cell or plant. It is well-known to the person skilled in the art what measures to take to obtain transcription and translation of a nucleic acid in a host cell or plant. In particular, generally the nucleic acid to be expressed is equipped with regulatory elements ensuring transcription and translation in the given host, i.e., elements which are recognized by the transcription and translation machinery of the chosen host. Such elements may include, e.g. promoters, terminators, enhancers, targeting signals, ribosome binding sites, etc. and are known in the art and also described herein below.

When used herein, the term "polypeptide" or "protein" (both terms are used interchangeably herein) means a peptide, a protein, or a polypeptide which encompasses amino acid chains of a given length, wherein the amino acid residues are linked by covalent peptide bonds. However, peptidomimetics of such proteins/polypeptides wherein amino acid(s) and/or peptide bond(s) have been replaced by functional analogs are also encompassed by the invention as well as other than the 20 gene-encoded amino acids, such as selenocysteine. Peptides, oligopeptides and proteins may be termed polypeptides. The terms polypeptide and protein are often used interchangeably herein. The term polypeptide also refers to, and does not exclude, modifications of the polypeptide, e.g., glycosylation, acetylation, phosphorylation and the like. Such modifications are well described in basic texts and in more detailed monographs, as well as in the research literature. The polypeptide (or protein) that is preferably meant herein is a wild-type or mutant CYCLOPS polypeptide.

Generally, the skilled person knows, because of his common general knowledge and the context when the term CYCLOPS is used, as to whether the nucleotide sequence or nucleic acid, or the amino acid sequence or polypeptide, respectively, is meant.

The term "position" when used in accordance with the present invention means the position of either an amino acid within an amino acid sequence depicted herein or the position of a nucleotide within a nucleic acid sequence depicted herein. The term "corresponding" as used herein also includes that a position is not only determined by the number of the preceding nucleotides/amino acids. The position of a given nucleotide in accordance with the present invention which may be substituted may vary due to deletions or additional nucleotides elsewhere in a (mutant or wild-type) *CYCLOPS* 5'-untranslated region (UTR) including the promoter and/or any other regulatory sequences or gene (including exons and introns). Similarly, the position of a given amino acid in accordance with the present invention which may be substituted may very due to deletion or addition of amino acids elsewhere in a (mutant or wild-type) CYCLOPS polypeptide.

Thus, under a "corresponding position" in accordance with the present invention it is preferably to be understood that nucleotides/amino acids may differ in the indicated number but may still have similar neighboring nucleotides/amino acids. Said nucleotides/amino acids which may be exchanged, deleted or added are also comprised by the term "corresponding position". Specifically, the skilled person may, when aligning the reference sequence (subject sequence) SEQ ID No:1 or 2 with a nucleotide or amino acid sequence of interest (query sequence), for example, inspect a sequence of interest for the sequence motif RXXS (or the corresponding nucleotide sequence encoding this motif, respectively) when looking for the amino acid position (or codon encoding the amino acid at said position) as specified herein (i.e., a position corresponding to position 50 and/or 154 of the amino acid sequence shown in SEQ ID No:2 or a position corresponding to position 148-150 and/or 460-462 of the nucleotide sequence shown in SEQ ID No:1, respectively). More specifically, the "S" of said motif is subject to phosphorylation by a DMI3 protein such as CCaMK. Said "S" is then replaced in a mutant CYCLOPS protein by an amino acid mimicking the effects of phosphorylation.

In order to determine whether a nucleotide residue or amino acid residue in a given (mutant or wild-type) CYCLOPS nucleotide/amino acid sequence corresponds to a certain position in the nucleotide sequence of SEQ ID No: 1 or the amino acid sequence of SEQ ID No: 2, the skilled person can use means and methods well-known in the art, e.g., alignments, either manually or by using computer programs such as BLAST2.0, which stands for Basic Local Alignment Search Tool or ClustalW or any other suitable program which is suitable to generate sequence alignments.

SEQ ID No: 1 is the nucleotide sequence encoding *Lotus japonicus* wild type CYCLOPS.
SEQ ID No: 2 is the *Lotus japonicus* wild-type amino acid sequence derived from SEQ ID No: 1. SEQ ID No: 3 is the nucleotide sequence encoding the *Lotus japonicus* double D mutant CYCLOPS and SEQ ID No:4 is the CYCLOPS double D mutant protein.

The following table provides an overview on the reference sequences used herein when the position of the point mutation in a nucleotide sequence or the substitution in an amino acid sequence is determined:

| SEQ ID No: | type of sequence | species |
|---|---|---|
| 1 | Wild-type nucleotide sequence | *L. japonicus* |
| 2 | Wild-type amino acid | *L. japonicus* |
| 3 | Double D nucleotide sequence | *L. japonicus* |
| 4 | Double D amino acid sequence | *L. japonicus* |

SEQ ID No:1:

```
atg gaa ggg agg ggg ttt tct ggt tta tat aga aac tca agt gaa gaa        48
Met Glu Gly Arg Gly Phe Ser Gly Leu Tyr Arg Asn Ser Ser Glu Glu
1               5                   10                  15

ttg ttc ctg aag aca gtg atg gag agc cct att ggt atg cca gtt cct        96
Leu Phe Leu Lys Thr Val Met Glu Ser Pro Ile Gly Met Pro Val Pro
                20                  25                  30

tca atg gag atg ctg ggt ttc aag aat gtt tct caa ggc ttt cgc gca       144
Ser Met Glu Met Leu Gly Phe Lys Asn Val Ser Gln Gly Phe Arg Ala
            35                  40                  45

gat agc gag gag ctt ttc aaa cgc tgg cta aca aat gga gag gga tac       192
Asp Ser Glu Glu Leu Phe Lys Arg Trp Leu Thr Asn Gly Glu Gly Tyr
        50                  55                  60

aat tca tca agc ata ggg ttt agc agt cga tta tca aag agg ata tcc       240
Asn Ser Ser Ser Ile Gly Phe Ser Ser Arg Leu Ser Lys Arg Ile Ser
65                  70                  75                  80

act gaa cta gtt aat gga tct aat cag cta caa gtt ggt gta gcc tca       288
Thr Glu Leu Val Asn Gly Ser Asn Gln Leu Gln Val Gly Val Ala Ser
                85                  90                  95
```

```
gat gga aga aac aat gac aaa cca ttc ata caa aat aac ctt ttg gca      336
Asp Gly Arg Asn Asn Asp Lys Pro Phe Ile Gln Asn Asn Leu Leu Ala
            100             105             110

aat gat gtt tca ggt gat ttc aat ttt cca atc aga gat cct gtt gat      384
Asn Asp Val Ser Gly Asp Phe Asn Phe Pro Ile Arg Asp Pro Val Asp
        115             120             125

aga gaa ctg caa cct agt aac ttg ttt cta gcc aag gcc tgg ttt ctc      432
Arg Glu Leu Gln Pro Ser Asn Leu Phe Leu Ala Lys Ala Trp Phe Leu
    130             135             140

agt gat caa cga atg aca aga agc cgg tcc tct gaa ttg cgg cgg cga      480
Ser Asp Gln Arg Met Thr Arg Ser Arg Ser Ser Glu Leu Arg Arg Arg
145             150             155             160

tat tct gaa atg caa aat ggt cta gcc aca caa gga ata gaa tcc att      528
Tyr Ser Glu Met Gln Asn Gly Leu Ala Thr Gln Gly Ile Glu Ser Ile
                165             170             175

tgc atg gat cct cag cat ggt gct gag gca aca aaa caa gaa gtt gca      576
Cys Met Asp Pro Gln His Gly Ala Glu Ala Thr Lys Gln Glu Val Ala
            180             185             190

aat ttc aat ggt tac aat tat ctc tct atg tgt gag ctt cca agt caa      624
Asn Phe Asn Gly Tyr Asn Tyr Leu Ser Met Cys Glu Leu Pro Ser Gln
        195             200             205

aag ggt tca ttc atg tct ccg tcc aac tca tgt tca tct aac ttc aac      672
Lys Gly Ser Phe Met Ser Pro Ser Asn Ser Cys Ser Ser Asn Phe Asn
    210             215             220

aca cct caa ttt ggc gac atg gat aaa gtt tca tct tgt gta agt atg      720
Thr Pro Gln Phe Gly Asp Met Asp Lys Val Ser Ser Cys Val Ser Met
225             230             235             240

ctg aaa ggg aca tta caa cgc cgg aga ctc agc agt caa ctt gag aaa      768
Leu Lys Gly Thr Leu Gln Arg Arg Arg Leu Ser Ser Gln Leu Glu Lys
                245             250             255

gaa gct gca gaa gat gac tta aat gga att ttt tat cct caa gaa cct      816
Glu Ala Ala Glu Asp Asp Leu Asn Gly Ile Phe Tyr Pro Gln Glu Pro
            260             265             270

ctt ttc caa act ggc ttt gat caa gga caa gaa aac tgg agt aat caa      864
Leu Phe Gln Thr Gly Phe Asp Gln Gly Gln Glu Asn Trp Ser Asn Gln
        275             280             285

acg cca gta aat gtt caa gta gac tct att ggt gaa gtt aag gat cat      912
Thr Pro Val Asn Val Gln Val Asp Ser Ile Gly Glu Val Lys Asp His
    290             295             300

gga gtc ctg caa aca cta gaa gga tcc aca aac cct gtc gtt gat ggt      960
Gly Val Leu Gln Thr Leu Glu Gly Ser Thr Asn Pro Val Val Asp Gly
305             310             315             320

ttt gca aat cag ata aac caa atc tat gtc gga aca gct tct gga gaa     1008
Phe Ala Asn Gln Ile Asn Gln Ile Tyr Val Gly Thr Ala Ser Gly Glu
                325             330             335

cct tct caa agt gaa tcc tct aat gct gca cca gta atc tcc tct ggt     1056
```

```
          Pro Ser Gln Ser Glu Ser Ser Asn Ala Ala Pro Val Ile Ser Ser Gly
                  340                 345                 350

tta gac aca tgc gaa ggt ccc ata aac tcg aat caa act ctc tgc gaa    1104
Leu Asp Thr Cys Glu Gly Pro Ile Asn Ser Asn Gln Thr Leu Cys Glu
        355                 360                 365

agc tca tgg aaa caa gta gga gtg agt aaa agt tca gaa aat act caa    1152
Ser Ser Trp Lys Gln Val Gly Val Ser Lys Ser Ser Glu Asn Thr Gln
        370                 375                 380

aat aga gtc aaa ggt ttc aga gaa cag atc atg gat aat ctg aaa gat    1200
Asn Arg Val Lys Gly Phe Arg Glu Gln Ile Met Asp Asn Leu Lys Asp
385                 390                 395                 400

gat aag aag aga aaa agt cta gaa aga tat gga tct ata aca tca gct    1248
Asp Lys Lys Arg Lys Ser Leu Glu Arg Tyr Gly Ser Ile Thr Ser Ala
                405                 410                 415

gtt tca gat gac aag gga gac acc act aaa aag cgt agg gtg gaa cgc    1296
Val Ser Asp Asp Lys Gly Asp Thr Thr Lys Lys Arg Arg Val Glu Arg
                420                 425                 430

tca agg aaa atg gct gaa gct aag gaa aga aat tcg aca cca tca gtt    1344
Ser Arg Lys Met Ala Glu Ala Lys Glu Arg Asn Ser Thr Pro Ser Val
                435                 440                 445

ccc tca gat atg caa gct gtc ttg aag cgg tgc gaa aac ctt gag aag    1392
Pro Ser Asp Met Gln Ala Val Leu Lys Arg Cys Glu Asn Leu Glu Lys
        450                 455                 460

gaa gtt cga tcg cta aaa ctc aac ttg tcc ttc atg aat aga aag gat    1440
Glu Val Arg Ser Leu Lys Leu Asn Leu Ser Phe Met Asn Arg Lys Asp
465                 470                 475                 480

tct gaa caa aca aag cag ata gaa gac ctt cag aag cag aat gaa gag    1488
Ser Glu Gln Thr Lys Gln Ile Glu Asp Leu Gln Lys Gln Asn Glu Glu
                485                 490                 495

ctg gca gat gaa aaa gag cgc ctc ctc gaa gag att gaa aga att cta    1536
Leu Ala Asp Glu Lys Glu Arg Leu Leu Glu Glu Ile Glu Arg Ile Leu
                500                 505                 510

tca gaa act gaa aaa atg taa                                        1557
Ser Glu Thr Glu Lys Met
        515
```

SEQ ID No.2:

```
Met Glu Gly Arg Gly Phe Ser Gly Leu Tyr Arg Asn Ser Ser Glu Glu
1               5                   10                  15

Leu Phe Leu Lys Thr Val Met Glu Ser Pro Ile Gly Met Pro Val Pro
            20                  25                  30

Ser Met Glu Met Leu Gly Phe Lys Asn Val Ser Gln Gly Phe Arg Ala
        35                  40                  45

Asp Ser Glu Glu Leu Phe Lys Arg Trp Leu Thr Asn Gly Glu Gly Tyr
        50                  55                  60

Asn Ser Ser Ser Ile Gly Phe Ser Ser Arg Leu Ser Lys Arg Ile Ser
65                  70                  75                  80

Thr Glu Leu Val Asn Gly Ser Asn Gln Leu Gln Val Gly Val Ala Ser
                85                  90                  95

Asp Gly Arg Asn Asn Asp Lys Pro Phe Ile Gln Asn Asn Leu Leu Ala
            100                 105                 110

Asn Asp Val Ser Gly Asp Phe Asn Phe Pro Ile Arg Asp Pro Val Asp
            115                 120                 125

Arg Glu Leu Gln Pro Ser Asn Leu Phe Leu Ala Lys Ala Trp Phe Leu
    130                 135                 140

Ser Asp Gln Arg Met Thr Arg Ser Arg Ser Ser Glu Leu Arg Arg Arg
145                 150                 155                 160

Tyr Ser Glu Met Gln Asn Gly Leu Ala Thr Gln Gly Ile Glu Ser Ile
                165                 170                 175

Cys Met Asp Pro Gln His Gly Ala Glu Ala Thr Lys Gln Glu Val Ala
            180                 185                 190

Asn Phe Asn Gly Tyr Asn Tyr Leu Ser Met Cys Glu Leu Pro Ser Gln
        195                 200                 205

Lys Gly Ser Phe Met Ser Pro Ser Asn Ser Cys Ser Ser Asn Phe Asn
    210                 215                 220

Thr Pro Gln Phe Gly Asp Met Asp Lys Val Ser Ser Cys Val Ser Met
225                 230                 235                 240

Leu Lys Gly Thr Leu Gln Arg Arg Arg Leu Ser Ser Gln Leu Glu Lys
            245                 250                 255

Glu Ala Ala Glu Asp Asp Leu Asn Gly Ile Phe Tyr Pro Gln Glu Pro
            260                 265                 270

Leu Phe Gln Thr Gly Phe Asp Gln Gly Gln Glu Asn Trp Ser Asn Gln
        275                 280                 285

Thr Pro Val Asn Val Gln Val Asp Ser Ile Gly Glu Val Lys Asp His
    290                 295                 300

Gly Val Leu Gln Thr Leu Glu Gly Ser Thr Asn Pro Val Val Asp Gly
305                 310                 315                 320
```

```
Phe Ala Asn Gln Ile Asn Gln Ile Tyr Val Gly Thr Ala Ser Gly Glu
            325             330             335

Pro Ser Gln Ser Glu Ser Ser Asn Ala Ala Pro Val Ile Ser Ser Gly
            340             345             350

Leu Asp Thr Cys Glu Gly Pro Ile Asn Ser Asn Gln Thr Leu Cys Glu
            355             360             365

Ser Ser Trp Lys Gln Val Gly Val Ser Lys Ser Ser Glu Asn Thr Gln
    370             375             380

Asn Arg Val Lys Gly Phe Arg Glu Gln Ile Met Asp Asn Leu Lys Asp
385             390             395             400

Asp Lys Lys Arg Lys Ser Leu Glu Arg Tyr Gly Ser Ile Thr Ser Ala
            405             410             415

Val Ser Asp Asp Lys Gly Asp Thr Thr Lys Lys Arg Arg Val Glu Arg
            420             425             430

Ser Arg Lys Met Ala Glu Ala Lys Glu Arg Asn Ser Thr Pro Ser Val
    435             440             445

Pro Ser Asp Met Gln Ala Val Leu Lys Arg Cys Glu Asn Leu Glu Lys
    450             455             460

Glu Val Arg Ser Leu Lys Leu Asn Leu Ser Phe Met Asn Arg Lys Asp
465             470             475             480

Ser Glu Gln Thr Lys Gln Ile Glu Asp Leu Gln Lys Gln Asn Glu Glu
            485             490             495

Leu Ala Asp Glu Lys Glu Arg Leu Leu Glu Glu Ile Glu Arg Ile Leu
            500             505             510

Ser Glu Thr Glu Lys Met
            515
```

In view of the difference between a wild-type CYCLOPS gene and a mutant CYCLOPS gene comprised by a plant of the present invention, the CYCLOPS gene comprised by a plant of the present invention may thus be regarded as a mutant/mutated CYCLOPS gene, mutant/mutated CYCLOPS allele, or variant CYCLOPS gene.
Thus, throughout the specification, the terms "mutant/mutated allele," "mutant/mutated *CYCLOPS* allele," "mutant/mutated *CYCLOPS* gene", or "variant *CYCLOPS* gene" are used interchangeably. Unless indicated otherwise herein, these terms refer to

(i) a nucleotide sequence which comprises at a first position corresponding to position 148-150 of the nucleotide sequence shown in SEQ ID No:1 and/or at a second position corresponding to position 460-462 of the nucleotide sequence shown in SEQ ID No:1 a codon encoding an amino acid mimicking the effects of phosphorylation, or
(ii) a nucleotide sequence that encodes a (mutant) CYCLOPS protein having at a first position corresponding to position 50 of the amino acid sequence of the CYCLOPS protein shown in SEQ ID No:2 and/or at a second position corresponding to position 154 of the amino acid sequence of the CYCLOPS protein shown in SEQ ID No:2 an amino acid mimicking the effects of phosphorylation.

Similarly, throughout the specification, the terms "mutant/mutated CYCLOPS protein/polypeptide," or "variant CYCLOPS protein/polypeptide" are used interchangeably and refer to

(i) a CYCLOPS protein having at a first position corresponding to position 50 of the amino acid sequence of the CYCLOPS protein shown in SEQ ID No:2 and/or at a second position corresponding to position 154 of the amino acid sequence of the CYCLOPS protein shown in SEQ ID No:2 an amino acid mimicking the effects of phosphorylation, or

(ii) a CYCLOPS protein encoded by a nucleotide sequence which comprises at a first position corresponding to position 148-150 of the nucleotide sequence shown in SEQ ID No:1 and/or at a second position corresponding to position 460-462 of the nucleotide sequence shown in SEQ ID No:1 a codon encoding an amino acid mimicking the effects of phosphorylation.

In contrast, unless indicated otherwise, the terms "wild-type allele," "wild-type *CYCLOPS* allele," or "wild-type *CYCLOPS* gene" refer to

(i) a nucleotide sequence which does not comprise at a first position corresponding to position 148-150 of the nucleotide sequence shown in SEQ ID No:1 and/or at a second position corresponding to position 460-462 of the nucleotide sequence shown in SEQ ID No:1 a codon encoding an amino acid mimicking the effects of phosphorylation, or

(ii) a nucleotide sequence that encodes a CYCLOPS protein that does not have an amino acid mimicking the effects of phosphorylation, said amino acid being preferably serine, at a first position corresponding to position 50 of the amino acid sequence of the CYCLOPS protein shown in SEQ ID No:2 and/or at a second position corresponding to position 154 of the amino acid sequence of the CYCLOPS protein shown in SEQ ID No:2 an amino acid mimicking the effects of phosphorylation.

Preferably, a "wild-type *CYCLOPS* gene" has at a first position corresponding to position 148-150 of the nucleotide sequence shown in SEQ ID No:1 and/or at a second position corresponding to position 460-462 of the nucleotide sequence shown in SEQ ID No:1 a codon encoding an amino acid which is capable of being phosphorylated, such as serine, threonine or tryptophane, with tryptophane being less preferred.

Such a "wild-type allele," "wild-type CYCLOPS allele," or "wild-type CYCLOPS gene" may, or may not, comprise mutations, other than the mutation that causes the S50D and/or S154D substitution.

Similarly, throughout the specification, the terms "wild-type CYCLOPS protein/polypeptide," are used interchangeably and refer to

(i) a CYCLOPS protein which does not have at a first position corresponding to position 50 of the amino acid sequence of the CYCLOPS protein shown in SEQ ID No:2 and/or at a second position corresponding to position 154 of the amino acid sequence of the CYCLOPS protein shown in SEQ ID No:2 an amino acid mimicking the effects of phosphorylation, or

(ii) a CYCLOPS protein encoded by a nucleotide sequence which does not comprise at a first position corresponding to position 148-150 of the nucleotide sequence shown in SEQ ID No:1 and/or at a second position corresponding to position 460-462 of the nucleotide sequence shown in SEQ ID No:1 a codon encoding an amino acid mimicking the effects of phosphorylation.

Preferably, a wild-type CYCLOPS protein has at a first position corresponding to position 50 of the amino acid sequence of the CYCLOPS protein shown in SEQ ID No:2 and/or at a second position corresponding to position 154 of the amino acid sequence of the CYCLOPS protein shown in SEQ ID No:2 an amino acid which is capable of being phosphorylated, such as serine, threonine or tyrosine, with tyrosine being less preferred.

In essence, as regards the CYCLOPS gene, the only difference between a wild-type plant and a plant of the present invention is that at a position as specified herein (in particular at a first position corresponding to position 148-150 of the nucleotide sequence of a *CYCLOPS* (wild-type) gene shown in SEQ ID No:1and/or at a second position corresponding to position 460-462 of the nucleotide sequence of a CYCLOPS (wild-type) gene shown in SEQ ID No:1), a plant of the present invention comprises a codon encoding an amino acid which mimics the effects of phosphorylation.

Likewise, as regards the CYCLOPS protein, the only difference between a wild-type plant and a plant of the present invention is that at a position as specified herein (in particular at a first position corresponding to position 50 of the amino acid sequence of a CYCLOPS (wild-type) protein shown in SEQ ID No:2 and/or at a second position corresponding to position 154 of the amino acid sequence of the CYCLOPS (wild-type) protein shown in SEQ ID No:2, the plant of the present invention comprises a codon encoding an amino acid which mimics the effects of phosphorylation.

In accordance with the present invention the term "amino acid mimicking the effect(s) of phosphorylation" means an amino acid which has/exerts the same effect and/or has the same physico-chemical properties as a phosphorylated amino acid, preferably physiological effect, as an amino acid which is phosphorylated.

In the context of the present invention, phosphorylation of the amino acid that is then mimicked by another amino acid, for example, by an acidic amino acid (i.e., an amino acid having an acidic side chain), is preferably effected by a kinase, in the context of the present invention phosphorylation of an amino acid comprised by a CYCLOPS protein is preferably effected by CCaMK (i.e., a DMI3 ortholog), either *in vitro* or *in vivo,* with *in vivo* being preferred. Without being bound by theory, it is assumed that due to the negative charge of an acidic amino acid the negative charge of a phosphate group added to amino acids such as serine or threonine is resembled, thereby mimicking phosphorylation.

The amino acid(s) that is/are preferably phosphorylated by CCaMK is/are one or more serine(s) and/or threonine (s) comprised by a CYCLOPS protein, more preferably it is the serine residue in a CYCLOPS protein at a position corresponding to position 50 of the amino acid sequence shown in SEQ ID No:2 and/or the serine residue in a CYCLOPS protein at a position corresponding to position 154 of the amino acid sequence shown in SEQ ID No:2. The amino acid mimicking the effect(s) of phosphorylation is preferably homolog, variant or the like of aspartic acid or glutamic acid which can be used in chemical synthesis of proteins, more preferably it is an acidic amino acid, even more preferably it is aspartate (Asp or D) or glutamate (Glu or E). However, the amino acid at a position corresponding to position 50 and/or 154 of the amino acid sequence of SEQ ID No:2 is different from serine in a (mutant) CYCLOPS protein of the present invention.

Accordingly, in a preferred embodiment a (mutant) *CYCLOPS* gene of the present invention encodes a protein comprising a S50D, S50E, S154D, S154E, S50D and S154D, S50D and S154E, S50E and S154D or S50E and S154E substitution, collectively referred to herein as CYCLOPS substitution(s). A particularly preferred (mutant) CYCLOPS protein encoded by a *CYCLOPS* gene of the present invention comprises the S50D and S154D ("double D") substitution (sometimes also referred to herein as double D CYCLOPS (protein)).

Accordingly, in a particularly preferred embodiment a plant of present invention has in its genome the nucleotide sequence of a (mutated) CYCLOPS gene shown in SEQ ID No:3. The CYCLOPS gene shown in SEQ ID No:3 encodes the double D CYCLOPS protein shown in SEQ ID No:4.

Preferably, the S50D or S50E substitution is a result of an alteration of the codon at a position corresponding to position 148-150 of the nucleotide sequence shown in SEQ ID No: 1. More preferably, the S50D or S50E substitution is a result of an alteration of the codon at a position corresponding to position 148-150 of the nucleotide sequence shown in SEQ ID No: 1 so that said codon encodes an amino acid mimicking the effects of phosphorylation such as an acidic amino acid, more preferably aspartate or glutamate, respectively ("D" is encoded by the codon "GAC" or "GAT", "E" is encoded by the codon "GAG" or "GAA" at said position).

Preferably, the S154D or S154E substitution is a result of an alteration of the codon at a position corresponding to position 460-462 of the nucleotide sequence shown in SEQ ID No: 1. More preferably, the S154D or S154E substitution is a result of an alteration of the codon at a position corresponding to position 460-462 of the nucleotide sequence shown in SEQ ID No: 1 so that said codon encodes an amino acid mimicking the effects of phosphorylation such as an acidic amino acid, more preferably aspartate or glutamate, respectively ("D" is encoded by the codon "GAC" or "GAT", "E" is encoded by the codon "GAG" or "GAA" at said position).

When used in the context of the present invention, the term "CYCLOPS" refers to a nucleotide sequence encoding a nuclear-localized protein with a coiled-coil motif or a nuclear-localized protein with a coiled-coil motif encoded by said nucleotide sequence. Also, the term "CYCLOPS" when used herein can refer to a CYCLOPS protein. The skilled person will recognize because of the context in which said term is used whether it refers to a nucleotide sequence or an amino acid sequence (protein). Similarly, the skilled person will recognize because of the context in which said term is used whether it refers to a mutant or wild-type CYCLOPS nucleotide sequence or protein, respectively, or to both.

The CYCLOPS protein is encoded by a gene isolated by the present inventors from *Lotus japonicus* (Yano et al., cited herein). The CYCLOPS protein from *Lotus japonicus* is an ortholog of the *Medicago truncatula* IPD3 protein. The IPD3 protein was recently identified as interacting Protein of DMI 3 (the *M. truncatula* CCaMK ortholog). Accordingly, the term "CYCLOPS" comprises orthologs of the *Medicago truncatula* IPD3 protein or the *Lotus japonicus* CYCLOPS protein (sometimes also referred to herein as "IPD3 proteins" or "IPD3 class proteins"). Various IPD3 proteins are known from diverse plants spanning from Liverworths, Mosses, Hornworts, Lycophytes, Monilophytes, Gymnosperms and Angiosperms which are also encompassed by the term "CYCLOPS" when used herein (see column 4 of Table 1 Wang et al. (2010), New Phytologist 186, 514-525)as well as and corresponding mutant derivatives thereof, i.e. the IPD3 proteins disclosed in Wang et al. may be modified in accordance with the teaching of the present invention so as to comprise at a first position corresponding to position 50 and/or at a second position corresponding to position 154 of the amino acid sequence of SEQ ID No:2 an amino acid mimicking the effects of phosphorylation:

Table 1 Sequences of the three genes obtained or used in the study[1]

| Species | Doesn't Make Infections 1 (DMI1) | Doesn't Make Infections 3 (DMI3) | Interacting Protein of DMI3 (IPD3) | Voucher# (Herbarium) |
|---|---|---|---|---|
| **Liverworts** | | | | |
| Haplomitrium gibbsiae | FJ913205 | FJ913229 | FJ913199 | Engel & von Konrat 28341 (F) |
| Treubia lacunosa | FJ913206 | FJ913230 | FJ913200 | Engel & von Konrat 28345 (F) |
| Dumortiera hirsuta | FJ913208 | FJ913231 | FJ913191 | Qiu 06048 (MICH) |
| Lunularia cruciata | | FJ913233 (FJ913254)* | | Qiu 01036 (MICH) |
| Concephalum sp. | | FJ913234 (FJ913255)* | | Qiu 94096 (IND) |
| Pellia epiphylla | FJ913207 | FJ913232 | FJ913196 | QW06018 (MICH) |
| Trichocolea tomentella | FJ913209 | | FJ913192 | Qiu 06038 (MICH) |
| **Mosses** | | | | |
| Takakia lepidozioides | FJ913210 (FJ913225) | FJ913235 (FJ913256) | | X-D Li 010 (MICH) |
| Polytrichum juniperinum | FJ913211 | FJ913236 | | QW06012 (MICH) |
| Physcomitrella patens | XM_001755593 XM_001758311 | AY155462 | D5545208 | |
| Fissidens taxifolius | | FJ913239 | FJ913198 | QW06020 (MICH) |
| Mnium affine | | FJ913237 | | QW06014 (MICH) |
| Climacium dendroides | FJ913212 | FJ913238 | FJ913197 | QW06019 (MICH) |
| **Hornworts** | | | | |
| Anthoceros agrestis | FJ913213 (FJ913226) | | | Qiu 99112 (Z) |
| Phaeoceros laevis | FJ913215 | FJ913240 | | Qiu 06032 (MICH) |
| Megaceros aenigmaticus | FJ913214 | FJ913241 | FJ913204 | Qiu 06044 (MICH) |
| **Lycophytes** | | | | |
| Lycopodium digitatum | FJ913216 | FJ913242 | FJ913194 | Qiu 08001 (MICH) |
| Huperzia squarrosa | FJ913217 | | FJ913195 | Qiu 05001 (MICH) |
| Selaginella moellendorffii | gnlINov06_contig132.28 gnlINov06_contig187.13 | gnlIFeb06_Contig101.15 | gnlIFeb06_Contig8.14 | |
| **Monilophytes** | | | | |
| Botrychium dissectum | | FJ913244 | | Qiu 96214 (IND) |
| Osmunda regalis | | FJ913243 | | Qiu 02072 (MASS) |
| **Gymnosperms** | | | | |
| Cycas revoluta | FJ913218 | FJ913245 | FJ913202 | Qiu 94051 (IND) |
| Zamia sp. | | | FJ913203 | Qiu 09001 (MICH) |
| Ginkgo biloba | | FJ913246 | | Qiu 01001 (MASS) |
| **Angiosperms** | | | | |
| Amborella trichopoda | | FJ913247 (FJ913257) | | Qiu 97123 (IND) |
| Peltandra virginica | FJ913223 (no DMI1b) | | | Qiu 08006 (MICH) |
| Lilium longiflorum | | U24188* | | |
| Smilacina racemosa | FJ913219 (no DMI1b) | FJ913248 | | QW06007 (MICH) |
| Oryza sativa | NM_001051466 NM_001058400 | AC097175.2 | EF569223 | |
| Zea mays | DQ403198, DQ403197 | DQ403196 | | |
| Aquilegia formosa | DT727692+ DT727691, DR940038+ DT940037 | | | |
| Clematis virginiana | FJ913221 (no DMI1b) | | | Povilus 08002 (MICH) |
| Vitis vinifera | AM423798, CAO46310 | CU459288 | CU459357 | |
| Arabidopsis thaliana | NM_124375 (no DMI1b) | | | |
| Acer sp. | | FJ913250 | | QW06005 (MICH) |
| Brassica chinensis | FJ913220 (FJ913227)* | | | Qiu 94122 (IND) |
| Populus trichocarpa | e_gw1.86.49.1 e_gw1.XIX.2465.1 | LG_XI204389461204411174 | LG_III000810 | |
| Euphorbia milii | | FJ913249 (FJ913258)* | | Qiu 94056 (IND) |
| Cucurbita pepo | FJ913224* | | | Qiu 08009 (MICH) |
| Malus domestica | | Z38126* | | |
| Ricinus communis | EEF35956, EEF44668 | | | |
| Arachis hypogaea | | EU395429* | | |
| Cercis canadensis | FJ913222 (FJ913228)* | FJ913251 (FJ913259)* | | Qiu 94127 (IND) |

Table 1 (Continued)

| Species | Doesn't Make Infections 1 (DMI1) | Doesn't Make Infections 3 (DMI3) | Interacting Protein of DMI3 (IPD3) | Voucher# (Herbarium) |
|---|---|---|---|---|
| Lotus japonicus | AB162158, AB162157 | AM230792 | EF569221 | |
| Medicago truncatula | AY497771, FJ974130 | AY502066 | EF117279 | |
| Pisum sativum | AJ973194 (no DMI1b) | AY502067 | EF569222 | |
| Sesbania rostrata | | EU622875* | | |
| Lamium sp. | | FJ913252 (FJ913260)* | | Qiu 95019 (IND) |
| Coffea arabica | | FJ913253 (FJ913261) | | Qiu 96113 (IND) |
| Nicotiana tabacum | EF613118 | U70923*, U38446* | | |
| Petunia × hybrida | (no DMI1b) | EF592572 | | |

[1]All sequences were used in the combined three-gene analyses except those with an asterisk, which were used only in single-gene analyses. The accessions in parentheses contain intron/exon information. The sequences obtained from GenBank are shown in gray.

The classification of a protein as CYCLOPS protein is based on its overall identity with known CYCLOPS proteins (IPD3 orthologs as described herein), which are, for example, shown in column 4 of Table 1 of Wang et al. In addition, the person skilled in the art is readily in a position to determine whether a protein based on its overall identity belongs to the IPD3 class of proteins by using means and methods for generating sequence alignments or structure prediction for determining motifs of proteins, in particular in the coiled-coil domain, see below, for example, available at www.expasy.ch or http://www.isrec.isb-sib.ch/software/software.html.

As mentioned before, Figure S5

**C**

```
                              ****              Coiled Coil
     Haplomitrium   DPTKKRRVERQRKMAEAKGRGNVPAMPADLQAAAKRCDALEKEVRSLKLNLSF
     Treubia        .A..........-.-............P........V............-........
Lv   Dumortiera     ....................A.P........V........................
     Pellia         GA..................A.PL......I........................
     Trichocolea    GA.R................A.P....I...V....T..................
     Physcomitrella VA......D.H...T......FTS.G.P.....V..L.E...D....RMT...
Ms   Fissidens      .A......D............ATS.G.P.....M..LE.........RM....
     Climacium      .A..R...D............ATS.G.P.....M-.L..........RM....
     Lycopodium     .S.........K.M.......SY..M..S.....T....................
Ly   Huperzia       .L.........K.M.T.....SY.....T.....V.C.ES...............
     Selaginella    VS.........K.M.V.....SS.....S.M...I...S................
     Oryza          ...........S........E.SST.VI.S.I.VVL...ET..............
     Vitis          ...........S........E.NLT....S.M.SIL...ET..............
An   Populus        ...........S........E.NMT..I.S.M.SVL...EN..............
     Lotus          .T.........S........E.NST.SV.S.M..VL...EN..............
     Pisum          .T.........S........E.NLT.TI.S.M..VM...EN..............
     Medicago       .N.........S........E.NLT.TI.S.M..IL...EN..............
```

**Fig. S5** Alignment of conserved functional domains in the three mycorrhizal proteins DMI1 (A), DMI3 (B), and IPD3 (C). The amino acids with asterisks in IPD3 represent the nuclear localization signal. A dot represents an amino acid identical to the one in the top sequence. Abbreviations: An – angiosperms, Hw – hornworts, Lv – liverworts, Ly – lycophytes, Ms – mosses.

from the supporting informtaion pertaining to the publication of Wang et al., cited herein, shows the coiled-coil domain that it a highly conserved structural motif of IPD3 orthologs which are encompassed by the term "CYCLOPS". Accordingly, for example, by way of the coiled-coil domain the skilled person is additionally or alternatively in a position to assign whether a protein is a "CYCLOPS" protein of the present invention.

Generally, any of the (mutant) CYCLOPS proteins described herein preferably comprises at a first position corresponding to position 50 of the amino acid sequence of the CYCLOPS protein shown in SEQ ID No:2 and/or at a second position corresponding to position 154 of the amino acid sequence of the CYCLOPS protein shown in SEQ ID No:2 an amino acid mimicking the effects of phosphorylation. Accordingly, the CYCLOPS protein which comprises said amino acid mimicking the effects of phosphorylation may thus also be referred to herein as "mutated", "substituted", or "variant" CYCLOPS protein.

Said mutated, substituted, or variant CYCLOPS protein is encoded by a nucleotide sequence comprising at a first position corresponding to position 148-150 of the nucleotide sequence shown in SEQ ID No:1 and/or at a second position corresponding to position 460-462 of the nucleotide sequence shown in SEQ ID No:1 a codon encoding an amino acid mimicking the effects of phosphorylation.

The person skilled in the art can easily determine whether a known or unknown, e.g. newly isolated, gene (gene of interest) or protein (protein of interest) encodes or is a CYCLOPS protein, respectively, by testing whether said gene encodes or is protein of interest that has the activity of a CYCLOPS protein (CYCLOPS activity).

For example, an activity test is shown in the appended Examples herein below. In particular, it is tested whether a protein of interest can interact with CCaMK/DMI3, for example, with any one of the DMI3 proteins listed in column 3 of Table 1 of Wang et al. shown above, cited herein. The preferred method for testing interaction is a two-hybrid system, for example, the yeast two-hybrid system. Such an interaction is exemplified in Wang et al., cited herein (see Figure 3 of Wang et al. and Materials and Methods). In particular, Wang et al. tested DMI3 and IPD3 interaction by applying a two hybrid assay (note that DMI3 and IPD3 proteins interact, since IPD3 is a phosphorylation target of DMI3) and showed cross-species interaction. Because of the cross-species interaction between DMI3 and IPD3 proteins, it is reasonable to assume that plants of the present invention that contain and/or express a (mutant) CYCLOPS gene/protein described herein become capable of spontaneously forming nodules, in particular root nodules. Also the appended Examples describe a two-hybrid interaction assay which can be applied to test for CYCLOPS activity of a protein of interest.

CYCLOPS activity of a protein of interest can alternatively and/or additionally be tested by a complementation assay. For example, it can be tested whether an inactive (or inactivated) CYCLOPS mutant which is not capable of forming nodules, in particular root nodules, for example, a *Lotus japonicus* CYCLOPS mutant (as described in Yano et al., cited herein) can be complemented so as to then (again) form nodules, in particular root nodules. Similarly, a mutant CYCLOPS protein of the present invention may be expressed in a plant to test for spontaneous nodule, in particular root nodule formation in comparison to a plant which does not express said mutant CYCLOPS protein.

A preferred modification of the complementation test is a complementation test done in a CCaMK (DMI3) mutant, i.e., one which is not capable of forming root nodules. Such a mutant is, for example, described in Tirichine et al., cited herein. Specifically, the gene encoding the protein of interest is mutated such that it contains at a first position corresponding to position 148-150 of the nucleotide sequence shown in SEQ ID No:1 and/or at a second position corresponding to position 460-462 of the nucleotide sequence shown in SEQ ID No:1, a codon encoding an amino acid mimicking the effects of phosphorylation, preferably an acidic amino acid such as aspartate or glutamate. Such a mutated gene encoding a protein of interest is introduced in a CCaMK mutant and tested for spontaneous development of nodules , in particular root nodules.

In the preferred modification of the complementation test, it is preferred that the gene encoding the cytokinin receptor LHK1 is inactive or that it is not constitutively active, i.e., that the plant does not have a LHK1 gain-of-function mutation which is capable of spontaneous root nodule formation (organogenesis), see Tirichine et al. (2007), Science 315, 104-107.

In view of the above, a (mutant) CYCLOPS protein encoded by a nucleotide sequence of the present invention has preferably CYCLOPS activity. Said CYCLOPS activity can be tested as described before, for example, in a two hybrid assay with CCaMK (DMI3) and/or in a complementation assay. More preferably, activity of a (mutant) CYCLOPS protein includes preferably the capability of a plant to develop/form spontaneously root nodules, in particular root nodules (root nodule formation/organogenesis) upon expression of a (mutant) CYCLOPS nucleotide sequence as described herein. In particular, spontaneous root nodule formation in a plant occurs when a plant contains and/or expresses a gene encoding a (mutant) CYCLOPS protein of the present invention which has at a first position corresponding to position 50 of the amino acid sequence of the CYCLOPS protein shown in SEQ ID No:2 and/or at a second position corresponding to position 154 of the amino acid sequence of the CYCLOPS protein shown in SEQ ID No:2 an amino acid mimicking the effects of phosphorylation.

Preferably, spontaneous root nodule formation occurs in the absence of rhizobia and/or rhizobial signal molecules.

The term "organogenesis", as used herein, means a process by which nodules, preferably root nodules, are developed, preferably spontaneously developed, from meristematic centres. Preferably, the root nodules developed by the plants of the present invention containing and/or expressing a mutant CYCLOPS gene maintain an active apical meristem that produces new cells for growth over the life of the nodule. Alternatively, it is preferred that nodules lose meristematic activity shortly after initiation, thus growth is due to cell expansion resulting in mature nodules which are spherical in shape.

Within nodules, nitrogen gas from the atmosphere is converted into ammonia, which is then assimilated into amino acids, nucleotides, chlorophyll and other cellular constituents such as vitamins, flavones, and hormones. Their ability to fix gaseous nitrogen makes legumes an ideal agricultural organism as their requirement for nitrogen fertilizer is reduced. Indeed high nitrogen content blocks nodule development as there is no benefit for the plant of forming the symbiosis. The energy for splitting the nitrogen gas in the nodule comes from sugar that is translocated from the leaf (a product of photosynthesis). Malate as a breakdown product of sucrose is the direct carbon source for the bacteroid. Nitrogen fixation in the nodule is very oxygen sensitive. Legume nodules harbor an iron containing protein called leghaemoglobin, closely related to animal myoglobin, to facilitate the conversion of nitrogen gas to ammonia.

However, though it is preferred that spontaneous root nodule formation occurs in the absence of rhizobia and/or rhizobial signal molecules if a plant of the present invention contains and/or expresses a gene encoding a (mutant) CYCLOPS protein as described herein, this does not mean that root nodules formed by a plant of the present invention are infected and then colonized by arbuscular mycorrhiza and/or rhizobes. It merely means that nodules, in particular root nodules are spontaneously formed/develop by plants because of containing and/or expressing a (mutant) CYCLOPS protein as described herein. Thus, rhizobes and/or rhizobial signal molecules are preferably not required for nodule formation, in particular root nodule formation.

As described herein, a plant of the present invention comprises a nucleotide sequence of a (mutant) CYCLOPS gene encoding a CYCLOPS protein having at a first position corresponding to position 50 of the amino acid sequence of the CYCLOPS protein shown in SEQ ID No:2 and/or at a second position corresponding to position 154 of the amino acid sequence of the CYCLOPS protein shown in SEQ ID No:2 an amino acid mimicking the effects of phosphorylation. In a preferred embodiment, the (mutant) CYCLOPS protein has an amino acid sequence which is at least 30%, more preferably of at least 40%, even more preferably of at least 50%, particularly preferred at least 60%, even particularly preferred at least 70%, especially preferred at least 80% and even more particularly preferred at least 90% identical to the amino acid sequence of SEQ ID No:2 and which has at a first position corresponding to position 50 of the amino acid sequence shown in SEQ ID No: 2 and/or at a second position corresponding to position 154 of the amino acid sequence shown in SEQ ID No: 2 an amino acid mimicking the effects of phosphorylation. Preferably, such a (mutant) CYCLOPS protein has CYCLOPS activity as described herein.

"Percent (%) amino acid sequence identity" with respect to (mutant and wild-type) CYCLOPS amino acid sequences identified herein is defined as the percentage of amino acid residues in a candidate sequence(sequence of interest) that are identical with the amino acid residues in the CYCLOPS amino acid sequence shown in SEQ ID No:2 or SEQ ID No:4, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publically available computer software such as BLAST, ALIGN, or Megalign (DNASTAR) software. Those skilled in the art can determine appropriate parameters for measuring alignment, including any algorithms needed to achieve maximum alignment over the full length of the sequences being compared. The degree of identity is preferably over the entire length with the amino acid sequence of SEQ ID No:2 or SEQ ID No:4. For example, an amino acid sequence of interest is aligned with the amino acid sequence of SEQ ID No:2 or SEQ ID No:4 and the identical amino acids are determined.

In another aspect, the present invention relates to a nucleic acid comprising the nucleotide sequence of a (mutant) CYCLOPS gene, wherein said nucleotide sequence comprises at a first position corresponding to position 148-150 of the nucleotide sequence shown in SEQ ID No:1 and/or at a second position corresponding to position 460-463 of the nucleotide sequence shown in SEQ ID No:1 a codon encoding an amino acid mimicking the effects of phosphorylation. Accordingly, the nucleotide sequences of the present invention encode a CYCLOPS protein.

In a preferred embodiment, the (mutant) nucleic acid is selected from the group consisting of:

(a) a nucleotide sequence having at least 30% identity to the nucleotide sequence shown in SEQ ID No:1 and having at a first position corresponding to position 148-150 of the nucleotide sequence shown in SEQ ID No:1 and/or at a second position corresponding to position 460-462 of the nucleotide sequence shown in SEQ ID No:1 a codon encoding an amino acid mimicking the effects of phosphorylation, said nucleotide sequence encodes a CYCLOPS protein;
(b) a nucleotide sequence shown in SEQ ID No:3;
(c) a nucleotide sequence encoding a CYCLOPS protein having an amino acid sequence which is at least 30% identical to the amino acid sequence of SEQ ID No:2 and which has at a first position corresponding to position 50 of the amino acid sequence shown in SEQ ID No: 2 and/or at a second position corresponding to position 154 of the amino acid sequence shown in SEQ ID No: 2 an amino acid mimicking the effects of phosphorylation;
(d) a nucleotide sequence encoding the polypeptide shown in SEQ ID No:4; and
(e) a fragment of the nucleotide sequence of (a), (b), (c) or (d), wherein the nucleotide sequence comprised by said fragment has at a first position corresponding to position 148-150 of the nucleotide sequence shown in SEQ ID No: 1 and/or at a second position corresponding to position 460-462 of the nucleotide sequence shown in SEQ ID No: 1 a codon encoding an amino acid mimicking the effects of phosphorylation, said fragment encodes a CYCLOPS protein, or

a nucleotide sequence which is complementary to a nucleotide sequence of any one of (a), (b), (c), or (d).

In order to determine whether a nucleic acid sequence has a certain degree of identity to the nucleic acid sequence encoding a (mutant or wild-type) CYCLOPS protein of the present invention, the skilled person can use means and methods well-known in the art, e.g., alignments, either manually or by using computer programs such as those mentioned further down below in connection with the definition of the term "hybridization" and degrees of homology. For example, BLAST2.0, which stands for Basic Local Alignment Search Tool (Altschul, Nucl. Acids Res. 25 (1997), 3389-3402; Altschul, J. Mol. Evol. 36 (1993), 290-300; Altschul, J. Mol. Biol. 215 (1990), 403-410), can be used to search for local sequence alignments. BLAST produces alignments of both nucleotide and amino acid sequences to determine sequence similarity. Because of the local nature of the alignments, BLAST is especially useful in determining exact matches or in identifying similar sequences. The fundamental unit of BLAST algorithm output is the High-scoring Segment Pair (HSP). An HSP consists of two sequence fragments of arbitrary but equal lengths whose alignment is locally maximal and for which the alignment score meets or exceeds a threshold or cutoff score set by the user. The BLAST approach is to look for HSPs between a query sequence and a database sequence, to evaluate the statistical significance of any matches found, and to report only those matches which satisfy the user-selected threshold of significance. The parameter E establishes the statistically significant threshold for reporting database sequence matches. E is interpreted as the upper bound of the expected frequency of chance occurrence of an HSP (or set of HSPs) within the context of the entire database search. Any database sequence whose match satisfies E is reported in the program output. Analogous computer techniques using BLAST (Altschul (1997), cited herein; Altschul (1993), cited herein; Altschul (1990), cited herein) are used to search for identical or related molecules in nucleotide databases such as GenBank or EMBL. This analysis is much faster than multiple membrane-based hybridizations. In addition, the sensitivity of the computer search can be modified to determine whether any particular match is categorized as exact or similar. The basis of the search is the product score which is defined as:

$$\frac{\% \text{sequence identity } \times \% \text{ maximum BLAST score}}{100}$$

and it takes into account both the degree of similarity between two sequences and the length of the sequence match. For example, with a product score of 40, the match will be exact within a 1-2% error; and at 70, the match will be exact. Similar molecules are usually identified by selecting those which show product scores between 15 and 40, although lower scores may identify related molecules.

Also, in another preferred embodiment, the present invention relates to nucleic acids which hybridize to a nucleotide sequence encoding a (mutant) CYCLOPS protein described herein. Generally, hybridizing nucleic acids comprise a nucleotide sequence comprising a first codon encoding an amino acid mimicking the effects of phosphorylation at a position corresponding to position 148-150 of the nucleotide sequence of the CYCLOPS gene shown in SEQ ID No: 1 and/or at a second codon encoding an amino acid mimicking the effects of phosphorylation at a position corresponding to position 460-462 of the nucleotide sequence of the CYCLOPS gene shown in SEQ ID No: 1.

The term "hybridizes" as used in accordance with the present invention may relate to hybridizations under stringent or non-stringent conditions. If not further specified, the conditions are preferably non-stringent. Said hybridization conditions may be established according to conventional protocols described, for example, in Sambrook, Russell "Molecular Cloning, A Laboratory Manual", Cold Spring Harbor Laboratory, N.Y. (2001); Ausubel, "Current Protocols in Molecular Biology", Green Publishing Associates and Wiley Interscience, N.Y. (1989), or Higgins and Hames (Eds.) "Nucleic acid hybridization, a practical approach" IRL Press Oxford, Washington DC, (1985). The setting of conditions is well within the skill of the artisan and can be determined according to protocols described in the art. Thus, the detection of only specifically hybridizing sequences will usually require stringent hybridization and washing conditions such as 0.1xSSC, 0.1% SDS at 65°C. Non-stringent hybridization conditions for the detection of homologous or not exactly complementary sequences may be set at 6xSSC, 1% SDS at 65°C. As is well known, the length of the probe and the composition of the nucleic acid to be determined constitute further parameters of the hybridization conditions. Note that variations in the above conditions may be accomplished through the inclusion and/or substitution of alternate blocking reagents used to suppress background in hybridization experiments. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility. Hybridizing nucleic acid molecules also comprise fragments of the above described molecules. Such fragments may represent nucleic acid sequences which encode a CYCLOPS protein, and which have a length of at least 12 nucleotides, preferably at least 15, more preferably at least 18, more preferably of at least 21 nucleotides, more preferably at least 30 nucleotides, even more preferably at least 40 nucleotides and most preferably at least 60 nucleotides and comprise a nucleotide sequence comprising a first codon encoding an amino acid mimicking the effects of phosphorylation at a position corresponding to position 148-150 of the nucleotide sequence of the CYCLOPS gene shown in SEQ ID No: 1 and/or at a second codon encoding an amino acid mimicking the effects of phosphorylation at a position corresponding to position 460-462 of the nucleotide sequence of the CYCLOPS gene shown in SEQ ID No: 1. Furthermore, nucleic acid molecules which hybridize with any of the aforementioned nucleic acid molecules also include complementary fragments, derivatives and allelic variants of these molecules. Additionally, a hybridization complex refers to a complex between two nucleic acid sequences by virtue of the formation of hydrogen bonds between complementary G and C bases and between complementary A and T bases; these hydrogen bonds may be further stabilized by base stacking interactions. The two complementary nucleic acid sequences hydrogen bond in an antiparallel configuration. A hybridization complex may be formed in solution (e.g., Cot or Rot analysis) or between one nucleic acid sequence present in solution and another nucleic acid sequence immobilized on a solid support (e.g., membranes, filters, chips, pins or glass slides to which, e.g., cells have been fixed). The terms complementary or complementarity refer to the natural binding of polynucleotides under permissive salt and temperature conditions by base-pairing. For example, the sequence "A-G-T" binds to the complementary sequence "T-C-A". Complementarity between two single-stranded molecules may be "partial", in which only some of the nucleic acids bind, or it may be complete when total complementarity exists between single-stranded molecules. The degree of complementarity between nucleic acid strands has significant effects on the efficiency and strength of hybridization between nucleic acid strands. This is of particular importance in amplification reactions, which depend upon binding between nucleic acids strands.

The term "hybridizing sequences" preferably refers to sequences which display a sequence identity of at least 30 or 40%, preferably at least 50%, more preferably at least 60%, even more preferably at least 70%, particularly preferred at least 80%, more particularly preferred at least 90%, even more particularly preferred at least 95%, 96%, 97% or 98% and most preferably at least 99% identity with a nucleic acid sequence as described above encoding a (mutant) CYCLOPS protein. Preferably, hybridizing sequences comprise a nucleotide sequence comprising a first codon encoding an amino acid mimicking the effects of phosphorylation at a position corresponding to position 148-150 of the nucleotide sequence of the CYCLOPS gene shown in SEQ ID No: 1 and/or at a second codon encoding an amino acid mimicking the effects of phosphorylation at a position corresponding to position 460-462 of the nucleotide sequence of the CYCLOPS gene shown in SEQ ID No: 1.

Moreover, the term "hybridizing sequences" preferably refers to sequences encoding a CYCLOPS protein having a sequence identity of at least 30%, preferably at least 40%, more preferably at least 50%, even more preferably at least 60%, particularly preferred at least 70%, more particularly preferred at least 80%, even more particularly preferred at least 90%, 95% or 98% and most preferably at least 99% identity with an amino acid sequence of a CYCLOPS protein shown in SEQ ID No:2.

Preferably, hybridizing sequences encode a CYCLOPS protein having at a first position corresponding to position 50 of the amino acid sequence of the CYCLOPS protein shown in SEQ ID No:2 and/or at a second position corresponding to position 154 of the amino acid sequence of the CYCLOPS protein shown in SEQ ID No:2 an amino acid mimicking the effects of phosphorylation.

"Percent (%) nucleotide sequence identity" with respect to (mutant or wild-type) CYCLOPS nucleotide sequences identified herein is defined as the percentage of nucleotide residues in a candidate sequence (sequence of interest) that are identical with the nucleotide residues in the CYCLOPS nucleotide sequence shown in SEQ ID No:1 or 3 after aligning the sequences and introducing gaps, if necessary, to achieve the maximum percent sequence identity, and not considering any conservative substitutions as part of the sequence identity. Alignment for purposes of determining percent nucleotide sequence identity can be achieved in various ways that are within the skill in the art, for instance, using publically available computer software such as BLAST, ALIGN, or Megalign (DNASTAR) software. Those skilled in the art can determine appropriate parameters for measuring alignment, including any algorithms needed to achieve maximum alignment over the full length of the sequences being compared. The degree of identity is preferably over the entire length with the nucleotide sequence of SEQ ID No:1 or 3. For example, a nucleotide sequence of interest is aligned with the nucleotide sequence of SEQ ID No:1 or 3 and the identical nucleotide residues are determined.

Such a definition also applies to the complement of a test sequence. Preferably the described identity exists over a region that is at least about 15 to 25 nucleotides in length, more preferably, over a region that is about 50 to 100 nucleotides in length.

Nucleic acids which hybridize with the nucleic acids disclosed herein can for instance be isolated from genomic libraries or cDNA libraries of bacteria, fungi, plants or animals. Preferably, such nucleic acids are from plant origin. Preferably, nucleic acids which hybridize to the nucleotide sequence of SEQ ID No:1 or 3 are variants, analogs or paralogs of such a nucleotide sequence.

As used herein, the term "analogs" refers to two nucleic acids that have the same or similar function, but that have evolved separately in unrelated organisms. As used herein, the term "orthologs" refers to two nucleic acids from different species, but that have evolved from a common ancestral gene by speciation. Normally, orthologs encode polypeptides having the same or similar functions. As also used herein, the term "paralogs" refers to two nucleic acids that are related by duplication within a genome. Paralogs usually have different functions, but these functions may be related (Tatusov, R. L. et al., 1997, Science 278, 631-637).

Herein, fragments are understood to mean parts of the nucleotide sequences of the present invention which are long enough to encode a protein having (mutant) CYCLOPS activity, preferably showing the biological activity as described above and which comprise a first codon encoding an amino acid mimicking the effects of phosphorylation at a position corresponding to position 148-150 of the nucleotide sequence of the CYCLOPS gene shown in SEQ ID No: 1 and/or at a second codon encoding an amino acid mimicking the effects of phosphorylation at a position corresponding to position 460-462 of the nucleotide sequence of the *CYCLOPS* gene shown in SEQ ID No: 1. A fragment of a (mutant) *CYCLOPS* nucleotide sequence has preferably a length of at least 15, 30, 45, 60, 100, 150, 200, 250, 300, 350, 400, 450 or 500 nucleotides.

The nucleic acids comprising a nucleotide sequence described herein are preferably comprised and/or expressed by a plant of the present invention. Such nucleic acids are comprised by the term "CYCLOPS".

In a second aspect the present invention relates to a plant encoding a CYCLOPS protein having at a first position corresponding to position 50 of the amino acid sequence of the CYCLOPS protein shown in SEQ ID No:2 and/or at a second position corresponding to position 154 of the amino acid sequence of the CYCLOPS protein shown in SEQ ID No:2 an amino acid mimicking the effects of phosphorylation. Such a plant preferably contains and/or expresses a nucleic acid comprising a nucleotide sequence encoding a (mutated) CYCLOPS gene as described herein.

Likewise, a polypeptide (protein) or fragment thereof encoded by a nucleic acid described herein is a further aspect of the present invention. Such proteins are described in detail elsewhere herein and are encompassed by the term "CYCLOPS".

A fragment of a (mutant) CYCLOPS protein has a length of at least 10, 20, 30, 40, 50, 60, 70, 80, 90, 100, 120, 140, 160, 180, 200, 250 or 300 amino acids. Said fragment prefeably comprises an amino acid having at a first position corresponding to position 50 of the amino acid sequence of the CYCLOPS protein shown in SEQ ID No:2 and/or at a second position corresponding to position 154 of the amino acid sequence of the CYCLOPS protein shown in SEQ ID No:2 an amino acid mimicking the effects of phosphorylation.

The nucleic acids comprising a (mutant) nucleotide sequence described herein which are preferably comprised by a plant are preferably integrated (preferably stably integrated) in the genome of said plant. However, it is also envisaged that said nucleic acids are present extrachromosomally in said plant. Extrachromosomal presence may be desirable in case of transient expression.

Accordingly, when a (mutant) *CYCLOPS* nucleic acid as described herein is integrated into a plant of the present invention, the plant as a result of said integration may be transgenic or non-transgenic.

Thus, by a "non-transgenic plant" is preferably meant a plant lacking a heterologous nucleotide sequence in its genome in at least one, preferably in each endogenous CYCLOPS locus. Said non-transgenic plant, however, contains preferably the mutant *CYCLOPS* gene as described herein.

Accordingly, it is a preferred embodiment of the present invention that the plant is a non-transgenic plant. Such a non-transgenic plant comprises in the nucleotide sequence of at least one *CYCLOPS* gene in its endogenous gene locus, one codon encoding an amino acid mimicking the effects of phosphorylation at a position as specified herein or two codons encoding an amino acid mimicking the effects of phosphorylation at the positions as specified herein . By "CYCLOPS gene in its endogenous locus" it is meant that the CYCLOPS gene comprised by a plant of the present invention is - when compared to a wild-type plant - located in the same locus, i.e., the *CYCLOPS* gene is positioned (located) on the same chromosome in the same chromosomal context (organization) as it is positioned in a wild-type plant (i.e., without there being any human intervention so as to transfer or re-locate the CYCLOPS gene comprised by the plant of the present invention to another location such as to a chromosome or genomic locus (position) different from that where the *CYCLOPS* gene is naturally located). Accordingly, the same genome-specific satellite markers which surround a wild-type *CYCLOPS* gene also surround the *CYCLOPS* gene comprised by a plant of the present invention.

"Chromosomal context" means that a (mutant) *CYCLOPS* gene of a plant of the present invention is located on the same chromosome as it is in a corresponding wild-type plant at the same location as it is in a wild-type plant. Accordingly, the same genes as in a wild-type plant are adjacent to the 5'- and 3'-end of the *CYCLOPS* gene comprised by a plant of the present invention. Accordingly, the same nucleotide sequences which are adjacent to the 5'-and 3'-end of the wild-type *CYCLOPS* gene are adjacent to the 5'- and 3'-end of the CYCLOPS gene comprised by a plant of the present invention. The similarity of the chromosomal context between the (mutant) *CYCLOPS* gene comprised by a plant of the present invention and that of the *CYCLOPS* gene of a corresponding wild-type plant can, for example, be tested as follows:

genome-specific satellite markers which surround a wild-type *CYCLOPS* gene and the (mutated) *CYCLOPS* gene of the present invention can be used together with sequences from the *CYCLOPS* gene (except for the codon(s) at the position as specified herein which is/are different between the wild-type *CYCLOPS* gene and the mutant *CYCLOPS* gene comprised by a plant of the present invention) for primer design and subsequent nucleic acid amplification, whereby the amplification product will be identical between a wild-type plant and a plant of the present invention. These genome-specific satellite markers can also be used for a fluorescent in situ hybridization (FISH) in order to check the location of the *CYCLOPS* gene In view of the fact that the (mutated) *CYCLOPS* gene of the present invention is located at the same chromosome at the same specific location, the "staining pattern" in FISH of the chromosome on which the wild-type *CYCLOPS* gene is located will be identical to the staining pattern in FISH of the chromosome on which the (mutated) *CYCLOPS* gene of the present invention is located.

It is an alternatively preferred embodiment of the present invention that the plant is a transgenic plant. Accordingly, the present invention relates also to transgenic plant cells which contain a nucleic acid molecule as described herein encoding a (mutated) CYCLOPS protein, wherein the nucleic acid molecule is foreign to the transgenic plant cell. By "foreign" it is meant that the nucleic acid molecule is either heterologous with respect to the plant cell, this means derived from a cell or organism with a different genomic background, or is homologous with respect to the plant cell but located in a different genomic environment than the naturally occurring counterpart of said nucleic acid molecule. This means that, if the nucleic acid molecule is homologous with respect to the plant cell, it is not located in its natural location in the genome of said plant cell when stably integrated into the genome, in particular it is surrounded by different genes. In this case the nucleic acid molecule may be either under the control of its own promoter or under the control of a heterologous promoter.

Plants of the present invention can, for example, be produced by site directed mutagenesis methods including those methods disclosed, for example, in International Application PCT/US00/23457, or U.S. Patent Nos. 6,271,360, 6,479,292, and 7,094,606. In one embodiment, such methods may involve the use of oligonucleotide-directed gene repair to introduce point mutations into the host cell genome, and can involve the use of single-stranded oligonucleotides, such as gene repair oligonucleobases (See, U.S. Patent Nos. 6,870,075 and 5,565,350).

In some embodiments of gene repair oligonucleobase-based approaches, the Rapid Trait Development System™ (RTDS) gene repair technology can be used to generate the plants of the present invention. RTDS is based on altering a targeted gene by utilizing the cell's own gene repair system to specifically modify the gene sequence in situ and not insert foreign DNA and gene expression control sequences. This procedure effects a precise change in the genetic sequence while the rest of the genome is left unaltered. In contrast to conventional transgenic GMOs, there is no integration of foreign genetic material, nor is any foreign genetic material left in the plant. The RTDS that effects this change is a chemically synthesized oligonucleotide which may be composed of both DNA and modified RNA bases as well as other chemical moieties, and is designed to hybridize at the targeted gene location to create a mismatched base-pair(s). This mismatched base-pair acts as a signal to attract the cell's own natural gene repair system to that site and correct (replace, insert or delete) the designated nucleotide(s) within the gene. Once the correction process is complete the RTDS molecule is degraded and the now-modified or repaired gene is expressed under that gene's normal endogenous control mechanisms. Thus, RTDS is a site-specific gene modification system that is effective at making precise changes in a gene sequence without the incorporation of foreign genes or control sequences. RTDS is described in detail in Beetham et al. (1999), Proc. Natl. Acad. Sci. USA Vol. 96: 8774-8778; Zhu et al. (1999), Proc. Natl. Acad. Sci. USA Vol. 96, pp. 8768-8773, Zhu et al. (2000), Nat. Biotechnol. Vol. 18:555-558, Kochevenko and Willmitzer (2003), Plant Physiology, Vol. 132: 174-184; Okuzaki and Toriyama (2004), Plant Cell Rep 22:509-512 or Dong et al. (2006), Plant Cell Rep 25: 457-465.

A further alternative are single-stranded oligonucleotide mutational vectors (SSMOVs) which can be prepared to introduce mutations to the CYCLOPS coding sequence. SSMOVs can be prepared in accordance with International Patent Application PCT/USOO/23457 and U.S. Patent Nos. 6,271,360; 6,479,292; and 7,094,606. The sequence of the SSOMV can be based on the same principles as the mutational vectors described in U.S. Pat. Nos. 5,565,350; 5,731,181, 5,756,325; 5,871,984; 5,760,012; 5,888,983; 5,795,972; 5,780,296; 5,945,339; 6,004,804; and 6,010,907 and in International Publication Nos. WO 98/49350; WO 99/07865; WO 99/58723; WO 99/58702; and WO 99/40789. In one embodiment, the sequence of the SSOMV contains two regions that are homologous with the target sequence separated by a region that contains the desired genetic alteration, termed the mutator region. The mutator region can have a sequence that is the same length as the sequence that separates the homologous regions in the target sequence, but having a different sequence. Such a mutator region can cause a substitution. Alternatively, the homologous regions in the SSOMV can be contiguous to each other, while the regions in the target gene having the same sequence are separated by one, two or more nucleotides. Such a SSOMV causes a deletion from the target gene of the nucleotides that are absent from the SSOMV. Lastly, the sequence of the target gene that is identical to the homologous regions may be adjacent in the target gene but separated by one two or more nucleotides in the sequence of the SSOMV. Such an SSOMV causes an insertion in the sequence of target gene.

In some embodiments, the nucleotides of the SSOMV are deoxyribonucleotides that are linked by unmodified phosphodiester bonds except that the 5' terminal and/or 3' terminal internucleotide linkage or alternatively the two 5' terminal and/or 3' terminal internucleotide linkages can be a phosphorothioate or phosphoamidate. As used herein an internucleotide linkage is the linkage between nucleotides of the SSOMV and does not include the linkage between the 5' end nucleotide or 3' end nucleotide and a blocking substituent. In a one specific embodiment the length of the SSOMV is between 21 and 60 deoxynucleotides and the lengths of the homology regions are, accordingly, a total length of at least 20 deoxynucleotides and at least two homology regions should each have lengths of at least 8 deoxynucleotides.

The SSOMVs can be designed to be complementary to either the coding or the non-coding strand of the target gene. When the desired mutation is a substitution of a single base, it is preferred that both the mutator nucleotide be a pyrimidine. To the extent that is consistent with achieving the desired functional result it is preferred that both the mutator nucleotide and the targeted nucleotide in the complementary strand be pyrimidines. In one embodiment, the SSOMVs that encode transversion mutations, i.e., a C or T mutator nucleotide is mismatched, respectively, with a C or T nucleotide in the complementary strand. In addition to the oligodeoxynucleotide, the SSOMV can contain a 5' blocking substituent that is attached to the 5' terminal carbons through a linker. The chemistry of the linker can be any chemistry and is at least 6 atoms long, and the linker is preferably flexible. A variety of nontoxic substituents such as biotin, cholesterol or other steroids or a non-intercalating cationic fluorescent dye can be used. Examples of reagents to make SSOMV include the reagents sold as Cy3™ and Cy5™ by Glen Research, Sterling Va. (now GE Healthcare), which are blocked phosphoroamidites that upon incorporation into an oligonucleotide yield 3,3,3',3'-tetramethyl N,N'-isopropyl substituted indomonocarbocyanine and indodicarbocyanine dyes, respectively. In one embodiment, the reagent is Cy3. When the indocarbocyanine is N-oxyalkyl substituted it can be conveniently linked to the 5' terminal of the oligodeoxynucleotide through a phosphodiester bond with a 5' terminal phosphate. The chemistry of the dye linker between the dye and the oligodeoxynucleotide is not critical and is chosen for synthetic convenience. When the commercially available Cy3 phosphoramidite is used as directed the resulting 5' modification consists of a blocking substituent and linker together which are a N- hydroxypropyl, N'-phosphatidylpropyl 3,3,3',3'-tetramethyl indomonocarbocyanine. In one embodiment the indocarbocyanine dye is tetra substituted at the 3 and 3' positions of the indole rings. Without limitations as to theory these substitutions prevent the dye from being an intercalating dye. The identity of the substituents at these positions is not critical. The SSOMV can, in addition, have a 3' blocking substituent. Again the chemistry of the 3' blocking substituent is not critical.

The term "introduction" or "transformation" as referred to herein encompasses the transfer of an exogenous nucleic acid into a host cell, irrespective of the method used for transfer.

Plant tissue capable of subsequent clonal propagation, whether by organogenesis or embryogenesis, may be transformed with a genetic construct of the present invention and a whole plant regenerated there from. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell may then be used to regenerate a transformed plant in a manner known to persons skilled in the art.

The transfer of foreign genes into the genome of a plant is called transformation. Transformation of plant species is now a fairly routine technique. Advantageously, any of several transformation methods may be used to introduce the gene of interest into a suitable ancestor cell. The methods described for the transformation and regeneration of plants from plant tissues or plant cells may be utilized for transient or for stable transformation. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al., (1982) Nature 296, 72- 74; Negrutiu I et al. (1987) Plant Mol Biol 8: 363-373); electroporation of protoplasts (Shillito R. D. et al. (1985) Bio/Technol 3, 1099-1 102); microinjection into plant material (Crossway A et al., (1986) Mol. Gen Genet 202: 179-185); DNA or RNA-coated particle bombardment (Klein TM et al., (1987) Nature 327: 70) infection with (non-integrative) viruses and the like. Transgenic plants, including transgenic crop plants, are preferably produced via Agrobacterium-mediated transformation. An advantageous transformation method is the transformation in planta. To this end, it is possible, for example, to allow the agrobacteria to act on plant seeds or to inoculate the plant meristem with agrobacteria. It has proved particularly expedient in accordance with the invention to allow a suspension of transformed agrobacteria to act on the intact plant or at least on the flower primordia. The plant is subsequently grown on until the seeds of the treated plant are obtained (Clough and Bent, Plant J. (1998) 16, 735- 743). Methods for Agrobacterium-mediated transformation of rice include well known methods for rice transformation, such as those described in any of the following: European patent application EP 1198985 A1 , Aldemita and Hodges (Planta 199: 612-617, 1996); Chan et al. (Plant Mol Biol 22 (3): 491-506, 1993), Hiei et al. (Plant J 6 (2): 271-282, 1994), which disclosures are incorporated by reference herein as if fully set forth. In the case of corn transformation, the preferred method is as described in either Ishida et al. (Nat. Biotechnol 14(6): 745-50, 1996) or Frame et al. (Plant Physiol 129(1): 13-22, 2002), which disclosures are incorporated by reference herein as if fully set forth. Said methods are further described by way of example in B. Jenes et al., Techniques for Gene Transfer, in: Transgenic Plants, Vol. 1, Engineering and Utilization, eds. S. D. Kung and R. Wu, Academic Press (1993) 128-143 and in Potrykus (Annu. Rev. Plant Physiol. Plant Molec. Biol. 42 (1991) 205-225). The nucleic acids or the construct to be expressed is preferably cloned into a vector, which is suitable for transforming Agrobacterium tumefaciens, for example pBin19 (Bevan et al., Nucl. Acids Res. 12 (1984) 8711). Agrobacteria transformed by such a vector can then be used in known manner for the transformation of plants, such as plants used as a model, like Arabidopsis {Arabidopsis thaliana is within the scope of the present invention not considered as a crop plant), or crop plants such as, by way of example, tobacco plants, for example by immersing bruised leaves or chopped leaves in an agrobacterial solution and then culturing them in suitable media. The transformation of plants by means of Agrobacterium tumefaciens is described, for example, by Hofgen and Willmitzer in Nucl. Acid Res. (1988) 16, 9877 or is known inter alia from F. F. White, Vectors for Gene Transfer in Higher Plants; in Transgenic Plants, Vol. 1, Engineering and Utilization, eds. S. D. Kung and R. Wu, Academic Press, 1993, pp. 15-38.

In addition to the transformation of somatic cells, which then have to be regenerated into intact plants, it is also possible to transform the cells of plant meristems and in particular those cells which develop into gametes. In this case, the transformed gametes follow the natural plant development, giving rise to transgenic plants. Thus, for example, seeds of Arabidopsis are treated with agrobacteria and seeds are obtained from the developing plants of which a certain proportion is transformed and thus transgenic (Feldman, KA and Marks MD (1987). Mol Gen Genet 208:274-289; Feldmann K (1992). In: C Koncz, N-H Chua and J Shell, eds, Methods in Arabidopsis Research. Word Scientific, Singapore, pp. 274-289). Alternative methods are based on the repeated removal of the inflorescences and incubation of the excision site in the center of the rosette with transformed agrobacteria, whereby transformed seeds can likewise be obtained at a later point in time (Chang (1994). Plant J. 5: 551-558; Katavic (1994). Mol Gen Genet, 245: 363-370). However, an especially effective method is the vacuum infiltration method with its modifications such as the "floral dip" method. In the case of vacuum infiltration of Arabidopsis, intact plants under reduced pressure are treated with an agrobacterial suspension (Bechthold, N (1993). C R Acad Sci Paris Life Sci, 316: 1194-1199), while in the case of the "floral dip" method the developing floral tissue is incubated briefly with a surfactant- treated agrobacterial suspension (Clough, SJ and Bent AF (1998) The Plant J. 16, 735-743). A certain proportion of transgenic seeds are harvested in both cases, and these seeds can be distinguished from non-transgenic seeds by growing under the above-described selective conditions. In addition the stable transformation of plastids is of advantages because plastids are inherited maternally is most crops reducing or eliminating the risk of transgene flow through pollen. The transformation of the chloroplast genome is generally achieved by a process which has been schematically displayed in Klaus et al., 2004 (Nature Biotechnology 22 (2), 225-229). Briefly the sequences to be transformed are cloned together with a selectable marker gene between flanking sequences homologous to the chloroplast genome. These homologous flanking sequences direct site specific integration into the plastome. Plastidal transformation has been described for many different plant species and an overview is given in Bock (2001) Transgenic plastids in basic research and plant biotechnology. J Mol Biol. 2001 Sep 21; 312 (3):425-38 or Maliga, P (2003) Progress towards commercialization of plastid transformation technology. Trends Biotechnol. 21, 20-28. Further biotechnological progress has recently been reported in form of marker free plastid transformants, which can be produced by a transient co-integrated maker gene (Klaus et al., 2004, Nature Biotechnology 22(2), 225-229).

T-DNA activation tagging (Hayashi et al. Science (1992) 1350-1353), involves insertion of T-DNA, usually containing a promoter (may also be a translation enhancer or an intron), in the genomic region of the gene of interest or 10 kb up- or downstream of the coding region of a gene in a configuration such that the promoter directs expression of the targeted gene. Typically, regulation of expression of the targeted gene by its natural promoter is disrupted and the gene falls under the control of the newly introduced promoter. The promoter is typically embedded in a T-DNA. This T-DNA is randomly inserted into the plant genome, for example, through Agrobacterium infection and leads to modified expression of genes near the inserted T-DNA. The resulting transgenic plants show dominant phenotypes due to modified expression of genes close to the introduced promoter.

The term "TILLING" is an abbreviation of "Targeted Induced Local Lesions In Genomes" and refers to a mutagenesis technology useful to generate and/or identify nucleic acids encoding proteins with modified expression and/or activity. TILLING also allows selection of plants carrying such mutant variants. These mutant variants may exhibit modified expression, either in strength or in location or in timing (if the mutations affect the promoter for example). These mutant variants may exhibit higher activity than that exhibited by the gene in its natural form. TILLING combines high-density mutagenesis with high-throughput screening methods. The steps typically followed in TILLING are: (a) EMS mutagenesis (Redei GP and Koncz C (1992) In Methods in Arabidopsis Research, Koncz C, Chua NH, Schell J, eds. Singapore, World Scientific Publishing Co, pp. 16-82; Feldmann et al., (1994) In Meyerowitz EM, Somerville CR, eds, Arabidopsis. Cold Spring Harbor Laboratory Press, Cold Spring Harbor, NY, pp 137-172; Lightner J and Caspar T (1998) In J Martinez-Zapater, J Salinas, eds, Methods on Molecular Biology, Vol. 82. Humana Press, Totowa, NJ, pp 91-104); (b) DNA preparation and pooling of individuals; (c) PCR amplification of a region of interest; (d) denaturation and annealing to allow formation of heteroduplexes; (e) Digestion with the mismatch specific endonuclease CEL1, where the presence of a heteroduplex in a pool is detected as an extra peak in the gel or capilary electrophoresis; (f) identification of the mutant individual; and (g) sequencing of the mutant PCR product. Methods for TILLING are well known in the art (McCallum et al., (2000) Nat Biotechnol 18: 455-457; reviewed by Stemple (2004) Nat Rev Genet 5: 145-50).

Any of the plants of the present invention which contains and/or expresses a mutated CYCLOPS gene as described herein is preferably agronomically exploitable.

"Agronomically exploitable" means that the plants of the present invention are useful for agronomical purposes. For example, the plants, though containing and/or expressing a (mutated) CYCLOPS gene should grow normally, preferably like a corresponding wild-type plant, serve for the purpose of being useful for crop production, fruit production, etc. A skilled person knows that, if a plant which contains and/or expresses a (mutated) CYCLOPS gene may not grow normally, appropriate measures such as backcrossing have to be taken.

In a further aspect, the present invention relates to harvestable parts or propagation material of the plant of the present invention.

The term "propagation material" comprises those components or parts of the plant which are suitable to produce offspring vegetatively or generatively. Suitable means for vegetative propagation are for instance cuttings, callus cultures, rhizomes or tubers. Other propagation material includes for instance fruits, seeds, seedlings, protoplasts, cell cultures etc. The preferred propagation materials are tubers and seeds. The invention also relates to harvestable parts of the plants of the invention such as, for instance, fruits, seeds, tubers, rootstocks, leaves, flowers, oil, meal.

In a preferred aspect the present invention any harvestable parts or material of a plant described herein contains up to 2%, 1%, 0.5%, 0.25%, 0.1%, 0.05%, 0.025%, 0.01%, 0.005% or 0.001 % (w/w) or (w/v) or (v/v), dependent on the form of said harvestable parts or material, of a (mutant) nucleotide sequence or fragment as described herein or a (mutant) amino acid sequence or fragment as described herein. Said mutant nucleotide sequence comprises comprises at least one (preferably two) of the codons at the position(s) as specified herein which encode an amino acid mimicking the effects of phosphorylation. Similarly, said mutant amino acid sequence comprises at least one (preferably) two of the amino acids at the positions as specified herein that mimic the effects of phosphorylation.

In a further aspect, the present invention relates to vectors, in particular plasmids, cosmids, viruses, bacteriophages and other vectors commonly used in genetic engineering, which contain the nucleic acids, prerefably the mutant nucleic acids of the present invention. In a preferred embodiment of the invention, the vectors of the invention are suitable for the transformation of fungal cells, cells of microorganisms such as yeast or bacterial cells, animal cells or, in particular, plant cells. Plant cell, as used herein includes, without limitation, algae, cyanobacteria, seeds, suspension cultures, embryos, meristematic regions, callus tissue, leaves, roots, shoots, gametophytes, sporophytes, pollen, and microspores. In a particularly preferred embodiment such vectors are suitable for the transformation of plants.

In a preferred embodiment, a (mutant) nucleic acid of the present invention which may preferably be comprised by a vector is operatively linked with an expression control sequence and/or with a termination signal sequence. Accordingly, a (mutant) nucleic acid of the present invention may preferably contain heterologous nucleotide sequences such as an expression control sequence and/or a termination signal sequence.

When such heterologous nucleotide sequences are incorporated into the genome of a plant, progeny of the plant can also comprise the heterologous nucleotide sequences. A progeny plant that comprises at least a portion of the heterologous nucleotide sequences of at least one progenitor transgenic plant is also a transgenic plant.

The term "heterologous" means that the nucleotide sequence either originates from a species different from that into which it is transformed or it may nevertheless originate from the same species, but be inserted at a location different from that where it originates from.

The term "operatively linked", as used in this context, refers to a functional linkage between one or more expression control sequences and the coding region of the heterologous nucleotide sequence such that the promoter sequence is able to initiate transcription of the gene of interest.

The term "expression" or "gene expression" means the transcription of a specific gene or specific genes or specific genetic construct. The term "expression" or "gene expression" in particular means the transcription of a gene or genes or genetic construct into structural RNA (rRNA, tRNA) or mRNA with or without subsequent translation of the latter into a protein. The process includes transcription of DNA and processing of the resulting mRNA product. Regulatory elements ensuring expression in plant cells are well known to those skilled in the art. They encompass promoters, enhancers, termination signals, targeting signals and the like. Examples are given further below in connection with explanations concerning vectors. In the case of eukaryotic cells, expression control sequences may comprise poly-A signals ensuring termination of transcription and stabilization of the transcript, for example, those of the 35S RNA from Cauliflower Mosaic Virus (CaMV) or the nopaline synthase gene from Agrobacterium tumefaciens. Additional regulatory elements may include transcriptional as well as translational enhancers. A plant translational enhancer often used is the CaMV omega sequences. Similarly, the inclusion of an intron (e.g. intron-1 from the shrunken gene of maize) has been shown to increase expression levels by up to 100-fold (Mait, Transgenic Research 6 (1997), 143-156; Ni, Plant Journal 7 (1995), 661-676).

The terms "regulatory element", "control sequence" and "promoter" are all used interchangeably herein and are to be taken in a broad context to refer to regulatory nucleic acid sequences capable of effecting expression of the sequences to which they are ligated. The term "promoter" typically refers to a nucleic acid control sequence located upstream from the transcriptional start of a gene and which is involved in recognising and binding of RNA polymerase and other proteins, thereby directing transcription of an operably linked nucleic acid. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, enhancers and silencers) which alter gene expression in response to developmental and/or external stimuli, or in a tissue-specific manner. Also included within the term is a transcriptional regulatory sequence of a classical prokaryotic gene, in which case it may include a -35 box sequence and/or -10 box transcriptional regulatory sequences. The term "regulatory element" also encompasses a synthetic fusion molecule or derivative that confers, activates or enhances expression of a nucleic acid molecule in a cell, tissue or organ.

A "plant promoter" comprises regulatory elements, which mediate the expression of a coding sequence segment in plant cells. Accordingly, a plant promoter need not be of plant origin, but may originate from viruses or micro-organisms, for example from viruses which attack plant cells. The "plant promoter" can also originate from a plant cell, e.g. from the plant which is transformed with the nucleic acid sequence to be expressed in the inventive process and described herein. This also applies to other "plant" regulatory signals, such as "plant" terminators. The promoters upstream of the nucleotide sequences useful in the methods of the present invention can be modified by one or more nucleotide substitution(s), insertion(s) and/or deletion(s) without interfering with the functionality or activity of either the promoters, the open reading frame (ORF) or the 3'-regulatory region such as terminators or other 3' regulatory regions which are located away from the ORF. It is furthermore possible that the activity of the promoters is increased by modification of their sequence, or that they are replaced completely by more active promoters, even promoters from heterologous organisms. For expression in plants, the nucleic acid molecule must, as described above, be linked operably to or comprise a suitable promoter which expresses the gene at the right point in time and with the required spatial expression pattern.

The promoter used may be a constitutive promoter or an organ-specific/tissue-specific promoter. A "constitutive promoter" refers to a promoter that is transcriptionally active during most, but not necessarily all, phases of growth and development and under most environmental conditions, in at least one cell, tissue or organ.

An organ-specific or tissue-specific promoter is one that is capable of preferentially initiating transcription in certain organs or tissues, such as the leaves, roots, seed tissue etc. For example, a "root-specific promoter" is a promoter that is transcriptionally active predominantly in plant roots, substantially to the exclusion of any other parts of a plant, whilst still allowing for any leaky expression in these other plant parts. Promoters able to initiate transcription in certain cells only are referred to herein as "cell-specific".

A seed-specific promoter is transcriptionally active predominantly in seed tissue, but not necessarily exclusively in seed tissue (in cases of leaky expression). The seed-specific promoter may be active during seed development and/or during germination. The seed specific promoter may be endosperm/aleurone/embryo specific. Examples of seed-specific promoters are known in the art. Further examples of seed-specific promoters are given in Qing Qu and Takaiwa (Plant Biotechnol. J. 2, 113-125, 2004).

A green tissue-specific promoter as defined herein is a promoter that is transcriptionally active predominantly in green tissue, substantially to the exclusion of any other parts of a plant, whilst still allowing for any leaky expression in these other plant parts.

Another example of a tissue-specific promoter is a meristem-specific promoter, which is transcriptionally active predominantly in meristematic tissue, substantially to the exclusion of any other parts of a plant, whilst still allowing for any leaky expression in these other plant parts.

The term "terminator" encompasses a control sequence which is a DNA sequence at the end of a transcriptional unit which signals 3' processing and polyadenylation of a primary transcript and termination of transcription. The terminator can be derived from the natural gene, from a variety of other plant genes, or from T-DNA. The terminator to be added may be derived from, for example, the nopaline synthase or octopine synthase genes, or alternatively from another plant gene.

A heterologous nucleotide sequence may also comprise a selectable marker. A selectable marker may be used for selecting plants transformed with a (mutant) nucleotide sequence as described herein.

Another embodiment of the invention relates to host cells, in particular prokaryotic or eukaryotic cells, genetically engineered with the (mutant) nucleic acids or vectors of the present invention or which are obtainable by a method for producing genetically engineered host cells, as well as to cells derived from such transformed host cells. Preferably, the host cell of the present invention is a bacterial, yeast, fungus, plant or animal cell, with a plant cell being particularly preferred.

In a further aspect, the present invention relates to a method for the production of a plant being capable developing nodules, preferably spontaneously developing root nodules, preferably in the absence of rhizobia and/or rhizobial signal molecules upon expressing the (mutant) nucleotide sequence of a nucleic acid molecule as described herein encoding a mutant CYCLOPS protein comprising the introduction of a nucleic acid or a vector as described herein into the genome of a plant, plant cell or plant tissue.

As a result of the afore described method, a plant cell is obtainable (obtained). Accordingly, a plant cell obtainable (obtained) according to the afore described method is yet another aspect of the present invention

In another aspect the present invention provides a method for the production of a plant being capable of spontaneously developing nodules, in particular root nodules, preferably in the absence of rhizobia and/or rhizobial signal molecules, in the absence of CCaMK activity (for example, because of a constitutively active CCaMK (DMI3 ortholog) and/or LHK1 activity (for example, because of a constitutively active LHK1 cytokinin receptor) comprising transforming a plant with a foreign nucleic acid molecule the presence or expression of which in the cells of said plant results in the spontaneous development of root nodules, preferably in the absence of rhizobia and/or rhizobial signal molecules.

Accordingly, the plant transformed with said foreign nucleic acid molecule does preferably not show constitutively active CCaMK activity and/or LHK1 activity.

Similarly, the foreign nucleic acid molecule does preferably not comprise a nucleotide sequence encoding a constitutively active CCaMK (DMI3 ortholog) and/or a nucleotide sequence encoding a constitutively active LHK1.

The foreign nucleic acid is preferably one described herein encoding a mutant CYCLOPS protein.

Further aspects of the present invention are:

- Use of a (mutant) nucleic acid molecule or the vector as described herein for the production of a plant, plant tissue or plant cell being capable of developing nodules, in particular root nodules, more preferably for developing spontaneously developing root nodules, preferably in the absence of rhizobia and/or rhizobial signal molecules.

- Uses of the plant and/or the harvestable parts or propagation material as described herein for the manufacture/breeding of plants.

- Uses of the plant and/or harvestable parts or propagation material derived thereof as described herein for the manufacture of a transgenic plant.

- Uses of the plant and/or the harvestable parts or propagation material derived thereof as described herein for the manufacture of plant material/products.

- Plant material/products obtainable (obtained) from a plant or harvestable parts thereof as described herein.

- Kit comprising a nucleic acid encoding a mutant CYCLOPS protein of the present invention, a vector comprising said nucleic acid and/or a plant of the present invention and rhizobia, preferably selected from at least one or more of the bacteria described herein after. Rhizobia fall into two classes of the proteobacteria - the alpha- and beta-proteobacteria. Alpha-proteobacteria include Rhizobiales. Rhizobiales include Bradyrhizobiaceae (B. sp.), Brucellaceae (Ochrobactrum sp.), Hyphomicrobiaceae (Azorhizobium sp., Devosia sp.), Methylobacteriaceae (Methylobacterium nodulans), Phyllobacteriaceae (Mesorhizobium sp., Phyllobacterium sp.), Rhizobiaceae (Rhizobium sp., Sinorhizobium sp.). Most of the rhizobia belong to the order Rhizobiales but several rhizobia occur in distinct bacterial orders of the proteobacteria. Rhizobiales include Bradyrhizobiaceae and Rhizobiaceae. Beta-proteobacteria include Burkholderiales. Burkholderiales include Burkholderiaceae (Burkholderia sp.) and Oxalobacteraceae (Herbaspirillum sp.).

- Use of at least one or more of rhizobial bacteria, preferably selected from at least one or more of those described above for the infection and/or colonization of a plant of the present invention, preferably of the root nodules formed by a plant of the present invention.

- An antibody specifically binding to a mutant CYCLOPS protein as described herein. Preferably, the antibody specifically binds to an epitope comprising the codon at a position corresponding to position 50 and/or 154 of the amino acid sequence of SEQ ID No:2, wherein said codon encodes an amino acid mimicking the effects of phosphorylation.

The invention may also be characterized by the following items:

1. A plant comprising a nucleotide sequence of a (mutant) CYCLOPS gene, wherein said nucleotide sequence comprises at a first position corresponding to position 148-150 of the nucleotide sequence shown in SEQ ID No: and/or at a second position corresponding to position 460-462 of the nucleotide sequence shown in SEQ ID No:1

a codon encoding an amino acid mimicking the effects of phosphorylation.

2. The plant of item 1, wherein said nucleotide sequence is integrated in the genome of said plant.

3. The plant of any one of the preceding items, wherein the nucleotide sequence of a CYCLOPS gene encodes a (mutant) CYCLOPS protein having an amino acid sequence which is at least 30% identical to the amino acid sequence of SEQ ID No:2 and which has at a first position corresponding to position 50 of the amino acid sequence shown in SEQ ID No: 2 and/or at a second position corresponding to position 154 of the amino acid sequence shown in SEQ ID No: 2 an amino acid mimicking the effects of phosphorylation.

4. The plant of any one of the preceding items, wherein the amino acid mimicking the effects of phosphorylation is an acidic amino acid, preferably gulamate or aspartate.

5. The plant of any one of the preceding items having in its genome the nucleotide sequence of a (mutated) CYCLOPS gene shown in SEQ ID No:3.

6. The plant of any one of the preceding items encoding the (mutated) CYCLOPS protein shown in SEQ ID No:4.

7. The plant of any one of the preceding items, wherein said plant is capable of developing root nodules, preferably spontaneously developing nodules, preferably in the absence of rhizobia and/or rhizobial signal molecules.

8. The plant of any one of the preceding items, which is a non-legume plant.

9. The plant of any one of the preceding items, which is a non-legume crop plant.

10. The plant of any one of the preceding items which is rice, barley, maize, oats, rye, sorghum, wheat and Poaceae grass, cucumber, tomato, melon or, wine.

11. The plant of item 1 or 2, which is non-transgenic.

12. The plant of item 11, wherein said plant comprises in the nucleotide sequence of at least one CYCLOPS gene in its endogenous gene locus, a first codon encoding an amino acid mimicking the effects of phosphorylation at a position corresponding to position 148-150 of the nucleotide sequence of the CYCLOPS gene shown in SEQ ID No: 1 and/or at a second codon encoding an amino acid mimicking the effects of phosphorylation at a position corresponding to position 460-462 of the nucleotide sequence of the CYCLOPS gene shown in SEQ ID No: 1.

13. Plant of any one of the preceding items, comprising a CYCLOPS protein having at a first position corresponding to position 50 of the amino acid sequence of the CYCLOPS protein shown in SEQ ID No:2 and/or at a second position corresponding to position 154 of the amino acid sequence of the CYCLOPS protein shown in SEQ ID No: 2 an amino acid mimicking the effects of phosphorylation.

14. Plant of any one of the preceding items, which is agronomical exploitable.

15. Harvestable parts or propagation material of the plant of any one of the preceding items.

16. A nucleic acid comprising the nucleotide sequence of a CYCLOPS gene, wherein said nucleotide sequence comprises at a first position corresponding to position 148-150 of the nucleotide sequence shown in SEQ ID No:1 and/or at a second position corresponding to position 460-463 of the nucleotide sequence shown in SEQ ID No:1 a codon encoding an amino acid mimicking the effects of phosphorylation.

17. The nucleic acid of item 16, wherein said nucleotide sequence is selected from the group consisting of:

(a) a nucleotide sequence having at least 30% identity to the nucleotide sequence shown in SEQ ID No: and having at a first position corresponding to position 148-150 of the nucleotide sequence shown in SEQ ID No:1 and/or at a second position corresponding to position 460-462 of the nucleotide sequence shown in SEQ ID No:1 a codon encoding an amino acid mimicking the effects of phosphorylation, said nucleotide sequence encodes a CYCLOPS protein;
(b) a nucleotide sequence shown in SEQ ID No:3;

(c) a nucleotide sequence encoding a CYCLOPS protein having an amino acid sequence which is at least 40% identical to the amino acid sequence of SEQ ID No:2 and which has at a first position corresponding to position 50 of the amino acid sequence shown in SEQ ID No: 2 and/or at a second position corresponding to position 154 of the amino acid sequence shown in SEQ ID No: 2 an amino acid mimicking the effects of phosphorylation;

(d) a nucleotide sequence encoding the polypeptide shown in SEQ ID No:4; and

(e) a fragment of the nucleotide sequence of (a), (b), (c) or (d), wherein the nucleotide sequence comprised by said fragment has at a first position corresponding to position 148-150 of the nucleotide sequence shown in SEQ ID No:1 and/or at a second position corresponding to position 460-462 of the nucleotide sequence shown in SEQ ID No:1 a codon encoding an amino acid mimicking the effects of phosphorylation, said fragment encodes a CYCLOPS protein, or

a nucleotide sequence which is complementary to a nucleotide sequence of any one of (a), (b), (c), or (d).

18. A vector comprising the nucleic acid molecule of item 16 or 17.

19. The vector of item 18, wherein said nucleic acid molecule is operatively linked with an expression control sequence.

20. The vector of item 18 or 19, wherein said nucleic acid molecule comprises at its 3'-end a termination signal sequence.

21. A (genetically engineered) host cell comprising the nucleic acid molecule of item 16 or 17 or the vector of any one of items 18-20.

22. The host cell of item 21, which is a plant cell.

23. A polypeptide encoded by the nucleic acid molecule of item 16 or 17.

24. A method for the production of a plant being capable developing root nodules, preferably spontaneously develop nodules in the absence of rhizobia upon expression of the nucleotide sequence as defined in item 16 or 17, comprising the introduction of the nucleic acid of item 16 or 17 or the vector of any one of items 18-20 into the genome of a plant, plant cell or plant tissue.

25. A plant cell obtainable according to the method of item 24.

26. A method for the production of a plant being capable of spontaneously developing nodules in the absence of rhizobia and/or rhizobial signal molecules and in the absence of CCAMK activity and/or LHK1 activity, comprising transforming a plant with a foreign nucleic acid molecule the presence or expression of which in the cells of said plant results in the spontaneous development of nodules in the absence of rhizobia.

27. Use of the nucleic acid molecule of item 16 or 17 or the vector of any one of items 18-20 for the production of a plant, plant tissue or plant cell being capable of spontaneously developing nodules in the absence of rhizobia.

28. Use of the plant of any one of the items 1-14 and/or the harvestable parts or propagation material of item 15 for the manufacture/breeding of plants.

29. Use of the plant of any one of items 1-14 and/or harvestable parts or propagation material derived thereof, for the manufacture of a transgenic plants.

30. Use of the plant of any one of items 1-14 and/or the harvestable parts or propagation material derived thereof, for the manufacture of plant material/products.

31. Plant material/products obtained from a plant of any one of items 1-14 or harvestable parts thereof.

32. Kit comprising the nucleic acid of item 16 or 17, the vector of any one of items 18-20 and/or a plant of any one of items 1-14 and one or more rhizobial bacteria, preferably selected from those described herein.

33. Use of one or more rhizobial bacteria for the infection and/or colonization of a plant of any one of items 1-14.

The Figures show:

### Figure 1

A) *In vitro* kinase assay of CCaMK and CYCLOPS. Phosphorylation of CYCLOPS by CCaMK was tested in the absence (EGTA) and presence of calcium, or calcium/calmodulin, respectively. Strongest phosphorylation was observed with calcium/calmodulin.

B) CYCLOPS protein sequence and *in vitro* phosphorylation sites (indicated in red), found by mass spectrometric analysis. The identified phosphorylated serine residues S14, S50, S154, S251 and S412 are located in the sequence motif RXX/S (indicated in green/red). Nuclear localization signals (NLS) and coiled coil domain are marked in bold and underlined.

### Figure 2

Effect of CYCLOPS phosphorylation site mutagenesis on symbiosis establishment. Wildtype *CYCLOPS* and phosphorylation site mutants *CYCLOPS-S50AS154A, CYCLOPS-S14AS154A* and *CYCLOPS-S50DS154D* were transformed into the *cyclops-3* mutant background by hairy root transformation. Composite plants were inoculated with M. *loti* and *G. intraradices,* respectively and symbiosis establishment was evaluated 5 wpi. Infection by *M. loti* / establishment of RNS was scored positive, if rhizobia infected nodules were observed (visualized by bacterial expression of the fluorescent marker protein DsRed). AM symbiosis was scored positive, if arbuscules were formed within root cortical cells (visualized by ink staining of AM structures). Transformed plants were identified by visually scoring the fluorescence of the transformation marker GFP. Scale bar M. *loti* inoculated plants 1 mm, Scale bar AM stain 200 $\mu$m.

### Figure 3

Detection of protein expression of HA-CYCLOPS and HA-CYCLOPS phosphorylation site mutants in M. *loti* infected hairy roots by Western blot analysis. Protein extracts from hairy roots were prepared 5 wpi with *M. loti* and probed with an anti-HA antibody directed against the HA-tag fused to CYCLOPS.

### Figure 4

CYCLOPS-S50DS154D induces spontaneous nodules independent of kinase active CCaMK. CYCLOPS-S50DS154D equipped with the endogenous promoter (*pcyclops*) was introduced into the *ccamk-3* mutant background and plants were inoculated with *Glomus intraradices,* or M. *loti* DsRed, while part of the plants was left un-inoculated. After 5 weeks, plants were scored for symbiosis formation. In all conditions tested, CYCLOPS-S50DS154D induced nodule organogenesis, but nodules were not infected with rhizobia. Further, AM was not formed. Scale bars 0.5 mm.

### Figure 5

Subcellular localization of CYCLOPS wildtype, CYCLOPS-S50DS154D and CYCLOPS-S50AS154A. TSapphire fusions to the carboxytermini of CYCLOPS wildtype, CYCLOPS-S50DS154D and CYCLOPS-S50AS154A were expressed in *N. benthamiana* and localization was assessed by confocal microscopy. All proteins showed exclusive nuclear localization. Scale bars 20 $\mu$m.

The invention will now be described by reference to the following Examples which are merely illustrative and are not construed as a limitation of the scope the present invention.

**Plasmid construction**

*CYCLOPS* genomic sequence was amplified by polymerase chain reaction (Sambrook *et al.,* cited herein) using primer "N-term sym30" (5'-CACCATGGAAGGGAGGGGG-3) (SEQ ID No:5) and "C-term sym30" (5'-TTACATTTTTTCAGTTTCTGATAGAATTC-3) (SEQ ID No:6) using Plasmid J18 (containing *Lotus japonicus* genomic *CYCLOPS* sequence, cloned from *Lotus japonicus* Gifu wildtype genomic DNA, (unpublished); www. http://www.kazusa.or.jp/lotus) and inserted into pENTR-D-TOPO vector by TOPO cloning, yielding plasmid pCEW415 (unpublished). 3xHA-tag was inserted upstream of, and in-frame with the start codon of the genomic *CYCLOPS* sequence, resulting in plasmid pCEW416 (unpublished). *CYCLOPS* native promoter was amplified with primers CEW7 (5'-CAC CCC AAA CTA TCA GGT CAA GTC TGC-3') (SEQ ID No:7) and CEW8 (5'-TCA AAG TCG ACG TTT GGC TCA ACA GCA CTT TC-3') (SEQ ID No:8) using J30 (unpublished) as template, containing native *CYCLOPS* promoter (2435 bp) cloned from *Lotus japonicus* Gifu wildtype genomic DNA). After removal of the 35S promoter, the *CYCLOPS* promoter was cloned into the binary vector pK7WG2D (Karimi et al. (2002), Trends Plant Sci 7, 193-195) yielding vector *"pCYCLOPSpromoterWG2D".*

In order to replace serine 50 of the CYCLOPS protein sequence by aspartate (corresponding to nucleotides 148-150 of the *CYCLOPS* coding sequence), whereby nucleotide triplet AGC encodes serine and GAC encodes aspartate), polymerase chain mutagenesis reaction was carried out using plasmid pCEW416 as template and primer pairs SY36 (5'-CTTTCGCGCAGAT<u>GAC</u>GAGGAGCTTTTC-3') (SEQ ID No:9) and SY37 (5'-GAAAAGCTCCTC<u>GTC</u>ATCT-GCGCGAAAG-3') (SEQ ID No:10) to generate mutation S50D, resulting in plasmid *pCEVV416-S50D.* After verification of the mutation by sequencing, a second polymerase chain mutagenesis reaction was carried out on template *pCEW416-S50D* to generate S154D (corresponding to nucleotides 460-462 of the *CYCLOPS* coding sequence), whereby nucleotide triplet TCC encodes serine and GAC encodes aspartate) by using primer pairs SY42 (5'-GACAAGAAGCCGGG<u>AC</u>TCT-GAATTGCGGTAC-3') (SEQ ID No:11) and SY43 (5'-GTACCGCAATTCAGA<u>GT</u>CCCGGCTTCTTGTC-3') (SEQ ID No: 12). The introduced mutation was confirmed by sequencing. The resulting plasmid was named *pCEVV416-S50DS154D.* The mutated genomic *CYCLOPS* sequence *3xHA-CYCLOPS-S50DS154D* was then recombined into the plasmid *pCYCLOPSpromoterWG2D* by LR reaction resulting in the vector *pCYCLOPS:HAgCYCLOPS-S50DS154D.* Correct orientation (3' to the *CYCLOPS* promoter) and correct nucleotide sequence of S50D and S154D mutated genomic *CYCLOPS* was verified by sequencing of *pCYCLOPS:HAgCYCLOPS-S50DS154D.*

**Generation of transgenic roots by hairy root transformation and evaluation of symbiosis formation, or spontaneous nodule organogenesis, respectively.**

*pCYCLOPS:HAgCYCLOPS-S50DS154D* was transformed into *A. Rhizogenes* strain AR1193 (Stougaard et al. (1987), Mol Gen Genet 207, 251-255) by electroporation. Transformation of *pCYCLOPS:HAgCYCLOPS-S50DS154D* into AR1193 was verified by colony PCR. In order to examine the effect of expression of *pCYCLOPS:gCYCLOPS-S50DS154D in planta,* the construct was transformed into the *L. japonicus cyclops-3* and *ccamk-3* mutant background (Perry et al. (2009), Plant Physiol 151, 1281-1291) by hairy root transformation (Diaz et al. (2005), In: Lotus japonicus Handbook. Marquez AJ, editor. Dordrecht, The Netherlands: Springer, 261-277.

Transformation of roots was verified by visualizing the transformation marker GFP. Transformed roots were either, incubated sterile for 8 weeks post transformation, inoculated for 5 weeks with *Mesorhizobium loti* Maff303099 expressing Dsred fluorescent protein (Maekawa et al. (2009), Plant J. 58, 183-194), or inoculated for 5 weeks with *Glomus intraradices.* Restoration of root nodule symbiosis in transformed mutant plant roots by *M. loti* was scored positive, if infected nodules (visualized by DsRed expressing M. *loti* bacteria) were observed. Restoration of AM symbiosis was scored positive, if the formation of arbuscules was observed in ink stained (Vierheilig et al. (1998), Appl Environ Microbiol 64, 5004-5007) root cortical cells. Formation of spontaneous nodules was scored positive, if uninfected nodules were formed on plant roots incubated under sterile conditions.

**In vitro kinase assay**

Phosphorylation of CYCLOPS by CCaMK was tested in the absence (EGTA) and presence of calcium, or calcium/calmodulin, respectively. Strongest phosphorylation was observed with calcium/calmodulin (see Figure 1). The assay was done in accordance with Yano et al., cited herein.

**Testing of CYCLOPS phosphorylation mutants**

Effect of CYCLOPS phosphorylation site mutagenesis on symbiosis establishment was done as follows. Wildtype *CYCLOPS* and phosphorylation site mutants *CYCLOPS-S50AS154A, CYCLOPS-S14AS154A* and *CYCLOPS-S50DS154D* were transformed into the *cyclops-3* mutant background by hairy root transformation. Composite plants were inoculated with M. *loti* and *G. intraradices,* respectively and symbiosis establishment was evaluated 5 wpi. Infection by *M. loti* / establishment of RNS was scored positive, if rhizobia infected nodules were observed (visualized by bacterial expression of the fluorescent marker protein DsRed). AM symbiosis was scored positive, if arbuscules were formed within root cortical cells (visualized by ink staining of AM structures). Transformed plants were identified by visually scoring the fluorescence of the transformation marker GFP (see Figure 2).

**Detection of CYCLOPS phosphorylation site mutants**

Detection of protein expression of HA-CYCLOPS and HA-CYCLOPS phosphorylation site mutants in *M. loti* infected hairy roots by Western blot analysis (see Yano et al., cited herein). Protein extracts from hairy roots were prepared 5 wpi with M. *loti* and probed with an anti-HA antibody directed against the HA-tag fused to CYCLOPS (see Figure 3).

**In planta experiments with phosphorylation site mutants**

CYCLOPS-S50DS154D induces spontaneous nodules independent of kinase active CCaMK. CYCLOPS-S50DS154D equipped with the endogenous promoter (*pcyclops*) was introduced into the *ccamk-3* mutant background and plants were inoculated with *Glomus intraradices,* or *M. loti* DsRed, while part of the plants was left un-inoculated. After 5 weeks, plants were scored for symbiosis formation. In all conditions tested, CYCLOPS-S50DS154D induced nodule organogenesis, but nodules were not infected with rhizobia. Further, AM was not formed (see Figure 4).

Subcellular localization experiments

Subcellular localization of CYCLOPS wildtype, CYCLOPS-S50DS154D and CYCLOPS-S50AS154A. TSapphire fusions to the carboxytermini of CYCLOPS wildtype, CYCLOPS-S50DS154D and CYCLOPS-S50AS154A were expressed in *N. benthamiana* and localization was assessed by confocal microscopy. All proteins showed exclusive nuclear localization (see Figure 5).

**Claims**

1. A plant comprising a nucleotide sequence of a (mutant) CYCLOPS gene, wherein said nucleotide sequence comprises at a first position corresponding to position 148-150 of the nucleotide sequence shown in SEQ ID No:1 and/or at a second position corresponding to position 460-462 of the nucleotide sequence shown in SEQ ID No:1 a codon encoding an amino acid mimicking the effects of phosphorylation.

2. The plant of any one of the preceding claims, wherein the nucleotide sequence of a CYCLOPS gene encodes a (mutant) CYCLOPS protein having an amino acid sequence which is at least 30% identical to the amino acid sequence of SEQ ID No:2 and which has at a first position corresponding to position 50 of the amino acid sequence shown in SEQ ID No: 2 and/or at a second position corresponding to position 154 of the amino acid sequence shown in SEQ ID No: 2 an amino acid mimicking the effects of phosphorylation.

3. The plant of any one of the preceding claims, wherein the amino acid mimicking the effects of phosphorylation is an acidic amino acid, preferably gulamate or aspartate.

4. The plant of any one of the preceding claims, wherein said plant is capable of developing root nodules, preferably spontaneously developing nodules, preferably in the absence of rhizobia and/or rhizobial signal molecules.

5. The plant of any one of the preceding claims which is rice, barley, maize, oats, rye, sorghum, wheat and Poaceae grass, cucumber, tomato, melon or, wine.

6. Plant of any one of the preceding claims, comprising a CYCLOPS protein having at a first position corresponding to position 50 of the amino acid sequence of the CYCLOPS protein shown in SEQ ID No:2 and/or at a second position corresponding to position 154 of the amino acid sequence of the CYCLOPS protein shown in SEQ ID No: 2 an amino acid mimicking the effects of phosphorylation.

7. Harvestable parts or propagation material of the plant of any one of the preceding claims.

8. A nucleic acid comprising the nucleotide sequence of a CYCLOPS gene, wherein said nucleotide sequence comprises at a first position corresponding to position 148-150 of the nucleotide sequence shown in SEQ ID No:1 and/or at a second position corresponding to position 460-463 of the nucleotide sequence shown in SEQ ID No:1 a codon encoding an amino acid mimicking the effects of phosphorylation.

9. The nucleic acid of claim 8, wherein said nucleotide sequence is selected from the group consisting of:

(a) a nucleotide sequence having at least 30% identity to the nucleotide sequence shown in SEQ ID No:1 and having at a first position corresponding to position 148-150 of the nucleotide sequence shown in SEQ ID No:1 and/or at a second position corresponding to position 460-462 of the nucleotide sequence shown in SEQ ID No:1 a codon encoding an amino acid mimicking the effects of phosphorylation, said nucleotide sequence encodes a CYCLOPS protein;
(b) a nucleotide sequence shown in SEQ ID No:3;
(c) a nucleotide sequence encoding a CYCLOPS protein having an amino acid sequence which is at least 40% identical to the amino acid sequence of SEQ ID No:2 and which has at a first position corresponding to position 50 of the amino acid sequence shown in SEQ ID No: 2 and/or at a second position corresponding to position 154 of the amino acid sequence shown in SEQ ID No: 2 an amino acid mimicking the effects of phosphorylation;
(d) a nucleotide sequence encoding the polypeptide shown in SEQ ID No:4; and
(e) a fragment of the nucleotide sequence of (a), (b), (c) or (d), wherein the nucleotide sequence comprised by said fragment has at a first position corresponding to position 148-150 of the nucleotide sequence shown in SEQ ID No:1 and/or at a second position corresponding to position 460-462 of the nucleotide sequence shown in SEQ ID No:1 a codon encoding an amino acid mimicking the effects of phosphorylation, said fragment encodes a CYCLOPS protein, or

a nucleotide sequence which is complementary to a nucleotide sequence of any one of (a), (b), (c), or (d).

10. A polypeptide encoded by the nucleic acid molecule of claim 8 or 9.

11. A method for the production of a plant being capable of spontaneously developing nodules in the absence of rhizobia and/or rhizobial signal molecules and in the absence of CCAMK activity and/or LHK1 activity, comprising transforming a plant with a foreign nucleic acid molecule the presence or expression of which in the cells of said plant results in the spontaneous development of nodules in the absence of rhizobia.

12. Use of the nucleic acid molecule of claim 8 or 9 or the vector comprising said nucleic acid molecule for the production of a plant, plant tissue or plant cell being capable of spontaneously developing nodules in the absence of rhizobia.

13. Use of the plant of any one of the claims 1-6 and/or the harvestable parts or propagation material of claim 7 for the manufacture/breeding of plants.

14. Use of the plant of any one of claims 1-6 and/or harvestable parts or propagation material derived thereof, for the manufacture of a transgenic plants.

15. Use of the plant of any one of claims 1-6 and/or the harvestable parts or propagation material derived thereof, for the manufacture of plant material/products.

# Figure 1

**A**

EGTA  Ca²⁺  Ca²⁺
CaM

autorad

—Cyclops-H
—CCaMK

coomassie

—Cyclops-H
—CCaMK

**B**

# Figure 2

**Figure 3**

# Figure 4

|  | Brightfield | GFP Marker | Brightfield |
| --- | --- | --- | --- |
| *ccamk-3 pCyclops: Cyclops S50DS154D* sterile | | | |
| *ccamk-3 pCyclops: Cyclops S50DS154D* AM | | | AM stain |
| *ccamk-3 pCyclops: Cyclops S50DS154D M. loti DsRed* | | | DsRed |

# Figure 5

CYCLOPS
TSapphire

CYCLOPS-S50DS154D
TSapphire

CYCLOPS-S50AS154A
TSapphire

Europäisches
Patentamt

European
Patent Office

Office européen
des brevets

## EUROPEAN SEARCH REPORT

**Application Number**

EP 10 17 5012

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2007/006318 A2 (UNIV AARHUS [DK]; TIRICHINE LEILA [DK]; JENSEN JENS STOUGAARD [DK]; SA) 18 January 2007 (2007-01-18) * figure 7; example 5 * | 7,11, 13-15 | INV. C12N15/82 A01H5/00 C12N15/29 |
| X | TIRICHINE LEILA ET AL: "A gain-of-function mutation in a cytokinin receptor triggers spontaneous root nodule organogenesis", SCIENCE (WASHINGTON D C), vol. 315, no. 5808, January 2007 (2007-01) , pages 104-107, XP002618633, ISSN: 0036-8075 * page 106, right-hand column, paragraph 2; figure 4 * * page 107, left-hand column * | 11 | |
| X | TIRICHINE LEILA ET AL: "Deregulation of a Ca2+/calmodulin-dependent kinase leads to spontaneous nodule development", NATURE (LONDON), vol. 441, no. 7097, June 2006 (2006-06), pages 1153-1156, XP002618634, ISSN: 0028-0836 * page 1154, left-hand column, paragraph 2 * * page 1156, left-hand column, last paragraph * | 11 | |

**TECHNICAL FIELDS SEARCHED (IPC)**

C12N
A01H
C07K

-/--

The present search report has been drawn up for all claims

| Place of search | Date of completion of the search | Examiner |
|---|---|---|
| The Hague | 27 January 2011 | Holtorf, Sönke |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

Europäisches
Patentamt

European
Patent Office

Office européen
des brevets

**EUROPEAN SEARCH REPORT**

Application Number

EP 10 17 5012

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GLEASON CYNTHIA ET AL: "Nodulation independent of rhizobia induced by a calcium-activated kinase lacking autoinhibition", NATURE (LONDON), vol. 441, no. 7097, June 2006 (2006-06), pages 1149-1152, XP002618635, ISSN: 0028-0836 * page 1150, right-hand column; figures 4 d,e * | 11 | |
| X | YANO KOJI ET AL: "CYCLOPS, a mediator of symbiotic intracellular accommodation", PROCEEDINGS OF THE NATIONAL ACADEMY OF SCIENCES OF THE UNITED STATES OF AMERICA, vol. 105, no. 51, December 2008 (2008-12), pages 20540-20545, XP002618636, ISSN: 0027-8424 * the whole document * | 11 | |
| A | CAPOEN WARD ET AL: "How CYCLOPS keeps an eye on plant symbiosis", PROCEEDINGS OF THE NATIONAL ACADEMY OF SCIENCES OF THE UNITED STATES OF AMERICA, vol. 105, no. 51, December 2008 (2008-12), pages 20053-20054, XP002618637, ISSN: 0027-8424 * page 20053, right-hand column; figure 1 * | 1 | TECHNICAL FIELDS SEARCHED (IPC) |
| A | FERGUSON BRETT J ET AL: "Molecular Analysis of Legume Nodule Development and Autoregulation", JOURNAL OF INTEGRATIVE PLANT BIOLOGY, vol. 52, no. 1, January 2010 (2010-01), pages 61-76, XP002618638, ISSN: 1672-9072 * page 66, left-hand column, paragraph 2 * | 1 | |

-/--

The present search report has been drawn up for all claims

| Place of search | Date of completion of the search | Examiner |
|---|---|---|
| The Hague | 27 January 2011 | Holtorf, Sönke |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 10 17 5012

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | HAYASHI TERUYUKI ET AL: "A dominant function of CCaMK in intracellular accommodation of bacterial and fungal endosymbionts", PLANT JOURNAL, vol. 63, no. 1, July 2010 (2010-07), pages 141-154, XP002618639, ISSN: 0960-7412 * page 144 - page 145, left-hand column * * page 150, left-hand column; figure 5 * | 1 | |
| A | GRIMSRUD PAUL A ET AL: "Large-Scale Phosphoprotein Analysis in Medicago truncatula Roots Provides Insight into in Vivo Kinase Activity in Legumes", PLANT PHYSIOLOGY (ROCKVILLE), vol. 152, no. 1, January 2010 (2010-01), pages 19-28, XP002618640, ISSN: 0032-0889 * page 24, right-hand column - page 25, left-hand column; figure 5 * | 1 | |

TECHNICAL FIELDS
SEARCHED (IPC)

The present search report has been drawn up for all claims

| Place of search | Date of completion of the search | Examiner |
|---|---|---|
| The Hague | 27 January 2011 | Holtorf, Sönke |

EPO FORM 1503 03.82 (P04C01)

**ANNEX TO THE EUROPEAN SEARCH REPORT**
**ON EUROPEAN PATENT APPLICATION NO.**                EP 10 17 5012

This annex lists the patent family members relating to the patent documents cited in the above-mentioned European search report.
The members are as contained in the European Patent Office EDP file on
The European Patent Office is in no way liable for these particulars which are merely given for the purpose of information.

27-01-2011

| Patent document cited in search report | Publication date | Patent family member(s) | Publication date |
|---|---|---|---|
| WO 2007006318 A2 | 18-01-2007 | EP 1902133 A2 | 26-03-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 0023457 W
- US 6271360 B
- US 6479292 B
- US 7094606 B
- US 6870075 B
- US 5565350 B
- US 5565350 A
- US 5731181 A
- US 5756325 A
- US 5871984 A
- US 5760012 A
- US 5888983 A
- US 5795972 A
- US 5780296594533960048044AND6010907 A
- WO 9849350 A
- WO 9907865 A
- WO 9958723 A
- WO 9958702 A
- WO 9940789 A
- EP 1198985 A1

### Non-patent literature cited in the description

- **RADUTOIU et al.** *Nature,* 2003, vol. 425, 585-592
- **MADSEN et al.** *Nature,* 2003, vol. 425, 637-640
- **OLDROYD ; DOWNIE.** *Nature Reviews,* 2004, vol. 5, 566-576
- **GROTH et al.** *Plant Cell,* 2010, vol. 22, 2509-2526
- **MARKMANN et al.** *PLoS Biol.,* 2008, vol. 6, e68
- **CHARPENTIER et al.** *Plant Cell,* 2009, vol. 20, 3467-3479
- **KANAMORI et al.** *Proc Natl Acad Sci USA,* 2006, vol. 103, 359-364
- **SAITO et al.** *Plant Cell,* 2007
- **TIRICHINE et al.** *Nature,* 2006, vol. 441, 1153-1156
- **YANO et al.** *Proc. Natl. Acad. Sci. USA,* 2008, vol. 105, 20540-20545
- **HAYASHI et al.** *Plant J.,* 2010, vol. 63, 141-154
- **MESSINESE et al.** *Mol Plant Microbe Interact.,* 2007, vol. 8, 912-921
- **GRIMSRUD et al.** *Plant Physiol.,* 2010, vol. 152, 19-28
- **WANG et al.** *New Phytologist,* 2010, vol. 186, 514-525
- **TIRICHINE et al.** *Science,* 2007, vol. 315, 104-107
- **ALTSCHUL.** *Nucl. Acids Res.,* 1997, vol. 25, 3389-3402
- **ALTSCHUL.** *J. Mol. Evol.,* 1993, vol. 36, 290-300
- **ALTSCHUL.** *J. Mol. Biol.,* 1990, vol. 215, 403-410
- **SAMBROOK ; RUSSELL.** Molecular Cloning, A Laboratory Manual. Cold Spring Harbor Laboratory, 2001
- **AUSUBEL.** Current Protocols in Molecular Biology. Green Publishing Associates and Wiley Interscience, 1989
- Nucleic acid hybridization, a practical approach. IRL Press, 1985
- **TATUSOV, R. L. et al.** *Science,* 1997, vol. 278, 631-637
- **BEETHAM et al.** *Proc. Natl. Acad. Sci. USA,* 1999, vol. 96, 8774-8778
- **ZHU et al.** *Proc. Natl. Acad. Sci. USA,* 1999, vol. 96, 8768-8773
- **ZHU et al.** *Nat. Biotechnol.,* 2000, vol. 18, 555-558
- **KOCHEVENKO ; WILLMITZER.** *Plant Physiology,* 2003, vol. 132, 174-184
- **OKUZAKI ; TORIYAMA.** *Plant Cell Rep,* 2004, vol. 22, 509-512
- **DONG et al.** *Plant Cell Rep,* 2006, vol. 25, 457-465
- **KRENS, F.A. et al.** *Nature,* 1982, vol. 296, 72-74
- **NEGRUTIU I et al.** *Plant Mol Biol,* 1987, vol. 8, 363-373
- **SHILLITO R. D. et al.** *Bio/Technol,* 1985, vol. 3, 1099-1 102
- **CROSSWAY A et al.** *Mol. Gen Genet,* 1986, vol. 202, 179-185
- **KLEIN TM et al.** *Nature,* 1987, vol. 327, 70

- **CLOUGH ; BENT.** *Plant J.,* 1998, vol. 16, 735-743
- **ALDEMITA ; HODGES.** *Planta,* 1996, vol. 199, 612-617
- **CHAN et al.** *Plant Mol Biol,* 1993, vol. 22 (3), 491-506
- **HIEI et al.** *Plant J,* 1994, vol. 6 (2), 271-282
- **ISHIDA et al.** *Nat. Biotechnol,* 1996, vol. 14 (6), 745-50
- **FRAME et al.** *Plant Physiol,* 2002, vol. 129 (1), 13-22
- Techniques for Gene Transfer. **B. JENES et al.** Transgenic Plants, Vol. 1 , Engineering and Utilization. Academic Press, 1993, vol. 1, 128-143
- **POTRYKUS.** *Annu. Rev. Plant Physiol. Plant Molec. Biol.,* 1991, vol. 42, 205-225
- **BEVAN et al.** *Nucl. Acids Res.,* 1984, vol. 12, 8711
- **HOFGEN ; WILLMITZER.** *Nucl. Acid Res.,* 1988, vol. 16, 9877
- Vectors for Gene Transfer in Higher Plants. **F. F. WHITE.** Transgenic Plants. Academic Press, 1993, vol. 1, 15-38
- **FELDMAN, KA ; MARKS MD.** *Mol Gen Genet,* 1987, vol. 208, 274-289
- **FELDMANN K.** Methods in Arabidopsis Research. Word Scientific, 1992, 274-289
- **CHANG.** *Plant J.,* 1994, vol. 5, 551-558
- **KATAVIC.** *Mol Gen Genet,* 1994, vol. 245, 363-370
- **BECHTHOLD, N.** *C R Acad Sci Paris Life Sci,* 1993, vol. 316, 1194-1199
- **CLOUGH, SJ ; BENT AF.** *The Plant J.,* 1998, vol. 16, 735-743
- **KLAUS et al.** *Nature Biotechnology,* 2004, vol. 22 (2), 225-229
- **BOCK.** Transgenic plastids in basic research and plant biotechnology. *J Mol Biol.,* 21 September 2001, vol. 312 (3), 425-38
- **MALIGA, P.** Progress towards commercialization of plastid transformation technology. *Trends Biotechnol.,* 2003, vol. 21, 20-28
- **HAYASHI et al.** *Science,* 1992, 1350-1353
- **REDEI GP ; KONCZ C.** Methods in Arabidopsis Research. Singapore, World Scientific Publishing Co, 1992, 16-82
- **FELDMANN et al.** Arabidopsis. Cold Spring Harbor Laboratory Press, 1994, 137-172
- **LIGHTNER J ; CASPAR T.** Methods on Molecular Biology. Humana Press, 1998, vol. 82, 91-104
- **MCCALLUM et al.** *Nat Biotechnol,* 2000, vol. 18, 455-457
- **STEMPLE.** *Nat Rev Genet,* 2004, vol. 5, 145-50
- **MAIT.** *Transgenic Research,* 1997, vol. 6, 143-156
- **NI.** *Plant Journal,* 1995, vol. 7, 661-676
- **QING QU ; TAKAIWA.** *Plant Biotechnol. J.,* 2004, vol. 2, 113-125
- **KARIMI et al.** *Trends Plant Sci,* 2002, vol. 7, 193-195
- **STOUGAARD et al.** *Mol Gen Genet,* 1987, vol. 207, 251-255
- **PERRY et al.** *Plant Physiol,* 2009, vol. 151, 1281-1291
- **DIAZ et al.** Lotus japonicus Handbook. Springer, 2005, 261-277
- **MAEKAWA et al.** *Plant J.,* 2009, vol. 58, 183-194
- **VIERHEILIG et al.** *Appl Environ Microbiol,* 1998, vol. 64, 5004-5007